# EUROPEAN PATENT APPLICATION

(11) **EP 4 328 494 A1**
(43) Date of publication of application: **28.02.2024**
(21) Application number: 22874142.7
(22) Date of filing: 28.03.2022
(51) Int. Cl.: F24C 15/20, G01N 15/06

(54) **OIL FUME SENSOR AND KITCHEN APPLIANCE**

(30) Priority: 29.09.2021 CN 202111149280; 29.09.2021 CN 202111149679; 29.09.2021 CN 202111149680; 29.09.2021 CN 202111149272
(71) Applicant: Foshan Shunde Midea Washing Appliances Manufacturing Co., Ltd., Foshan, Guangdong 528311 (CN)
(72) Inventor: LIU, Yulei, Foshan, Guangdong 528311 (CN); CHEN, Yinzhi, Foshan, Guangdong 528311 (CN); CHENG, Gang, Foshan, Guangdong 528311 (CN)
(74) Representative: RGTH
(86) International application number: PCT/CN2022/083470
(87) International publication number: WO 2023/050741

(57) **Abstract**

Provided are an oil fume sensor (100) and a kitchen appliance (1000). The oil fume sensor (100) includes a housing (10) having an accommodation cavity (11) and a sensor assembly (30) located in the accommodation cavity (11).

## Description

This application claims priorities to Chinese Patent Application No. 202111149680.X, No. 202111149280.9, No. 202111149679.7, and No. 202111149272.4, all filed on September 29, 2021, the entire disclosure of which are incorporated herein by reference.

### FIELD

The present disclosure relates to the field of household appliances, and more particularly, to an oil fume sensor and a kitchen appliance.

### BACKGROUND

An intelligent range hood is a range hood with a predetermined intelligent adjustment function. In order to ensure timely response of the intelligent range hood for oil fumes, oil fume sensors of various types are indispensable. The oil fume sensor typically detects oil fumes in a manner of detecting a concentrations of oil fume dust particles through light scattering. However, such a detection method for the oil fumes requires the oil fume sensor to be located at a position where the oil fumes flows. Therefore, the oil fume sensor is prone to being contaminated by the oil fumes, which may adversely affect performance and a service life of the oil fume sensor.

### SUMMARY

Embodiments of the present disclosure provide an oil fume sensor and a kitchen appliance.

According to an embodiment of the present disclosure, the oil fume sensor includes a housing having an accommodation cavity, and a sensor assembly located in the accommodation cavity.

With the oil fume sensor according to the embodiment of the present disclosure, the housing has a first side wall and a second side wall. The second side wall is tightly fitted to an outer side of the first side wall to allow the accommodation cavity to be formed into an enclosed space.

In some embodiments, the second side wall is in an interference fit with the outer side of the first side wall to allow for a tight fit between the second side wall and the outer side of the first side wall.

In some embodiments, the sensor assembly includes a main control board. The accommodation cavity includes a first receiving cavity, and the main control board is accommodated in the first receiving cavity. The housing includes an exhaust cover and an upper cover. The exhaust cover includes a first cover body and a first exhaust pipe penetrating the first cover body. The first exhaust pipe has a sealing groove formed at an end of the first exhaust pipe. The upper cover includes a second cover body and a second exhaust pipe penetrating the second cover body. The second exhaust pipe is provided with a sealing protrusion engaged in the sealing groove. An interior of the first exhaust pipe is in communication with an interior of the second exhaust pipe to form an exhaust cavity. The first receiving cavity is formed by the first cover body, the second cover body, the first exhaust pipe, and the second exhaust pipe.

In some embodiments, the first side wall is formed at an outer side wall of the sealing protrusion, and the second side wall is formed at an inner side wall of the sealing groove.

In some embodiments, the second side wall is formed at an inner side wall of the sealing protrusion, and the first side wall is formed at an outer side wall of the sealing groove.

In some embodiments, the upper cover is provided with a hollow protector protruding from the upper cover. The second side wall is formed at an inner edge of the protector. The main control board is provided with a socket protruding from the main control board. The first side wall is formed at a periphery of the socket.

In some embodiments, the sensor assembly includes a light emitter, a light receiver, and a lens. The accommodation cavity includes a second receiving cavity, and the light emitter and the light receiver are accommodated in the second receiving cavity. The housing includes an upper cover and a bottom housing. The bottom housing includes a first housing and a first air inlet pipe penetrating the first housing. The second receiving cavity is formed by the upper cover, the first housing, and the lens.

In some embodiments, the first side wall is formed at a peripheral wall of the upper cover. The first housing is provided with a protruding ring. The second side wall is formed at an inner side wall of the protruding ring.

In some embodiments, the housing further includes an exhaust cover. The exhaust cover has a receiving groove. The second side wall is formed at an inner side wall of the receiving groove. The first housing is provided with a protruding ring. The first side wall is formed at an outer side wall of the protruding ring.

In some embodiments, the housing further includes a fixing portion configured to restrict a position of the lens. The first side wall is formed at an outer side wall of the lens, and the second side wall is formed at an inner side wall of the fixing portion; and/or the second side wall is formed at an inner side wall of the lens, and the first side wall is formed at an outer side wall of the fixing portion.

According to the oil fume sensor and the kitchen appliance in the embodiments of the present disclosure, the second side wall is tightly fitted to the outer side of the first side wall to form the enclosed space, which prevents external oil fumes from easily entering the accommodation cavity. Therefore, contamination of the sensor assembly is further avoided or reduced. Moreover, performance and a service life of the oil fume sensor are ensured.

According to the oil fume sensor in the embodiments of the present disclosure, the housing further has an air inlet and an air outlet that are each in communication with the accommodation cavity. The housing includes a lower housing and an air inlet pipe penetrating the lower housing. The air inlet pipe internally has an air inlet cavity. The air inlet is formed at a position communicating between the air inlet cavity and the accommodation cavity. The air inlet cavity is tapered towards the air inlet.

In some embodiments, the air inlet cavity is in a truncated cone shape.

In some embodiments, the sensor assembly includes a light emitting assembly and a light receiving assembly. A mounting portion is disposed in the accommodation cavity. A second receiving cavity is formed by the mounting portion and the housing. The light emitting assembly and/or the light receiving assembly are mounted in the second receiving cavity. The housing has a pressurizing channel. The pressurizing channel has an end in communication with the second receiving cavity and another end in communication with an external environment.

In some embodiments, the lower housing includes a bottom housing and an air inlet housing. The second receiving cavity is formed by the bottom housing and the mounting portion. The air inlet pipe includes a first air inlet pipe and a second air inlet pipe. The first air inlet pipe penetrates the bottom housing, and the second air inlet pipe penetrates the air inlet housing. An interior of the first air inlet pipe is in communication with an interior of the second air inlet pipe to form the air inlet cavity. The bottom housing has an opening, and the air inlet housing has a third through hole. The opening is in communication with the third through hole to form the pressurizing channel.

In some embodiments, an oil reduction cavity is formed by an outer wall of the second air inlet pipe, an inner wall of the air inlet housing, and the bottom housing together. The opening and the third through hole are each in communication with the oil reduction cavity.

In some embodiments, the housing is provided with a plurality of fins. Each of the plurality of fins has a light-transmitting hole. The plurality of fins is arranged at intervals in the second receiving cavity along an optical axis of the light emitting assembly and/or an optical axis of the light receiving assembly.

In some embodiments, the housing further includes an upper housing and an exhaust pipe penetrating the upper housing. The accommodation cavity is formed by the upper housing and the lower housing together. The exhaust pipe has an exhaust cavity. The air outlet is at a position communicating between the exhaust cavity and the second receiving cavity.

In some embodiments, an axis of the air inlet cavity and an axis of the exhaust cavity are located on one straight line.

According to the oil fume sensor in the embodiments of the present disclosure, a detection cavity is formed at a position where the accommodation cavity contacts oil fumes. The detection cavity has a inclined guide surface. A through hole is formed at a lower cavity wall of the detection cavity. The inclined guide surface is configured to guide an oil drop to be discharged out of the detection cavity through the through hole.

In some embodiments, the through hole includes a first through hole. The housing has an air inlet and an air outlet. The air inlet and the air outlet are each in communication with the detection cavity. The inclined guide surface is formed at the lower cavity wall of the detection cavity. The air inlet serves as the first through hole.

In some embodiments, the through hole further includes a second through hole. The second through hole is formed at the lower cavity wall of the detection cavity and spaced apart from the first through hole.

In some embodiments, an inclination angle of the inclined guide surface relative to a horizontal plane is greater than or equal to 10°.

In some embodiments, the inclined guide surface includes a first inclined guide surface formed at an upper cavity wall of the detection cavity.

In some embodiments, the inclined guide surface includes a second inclined guide surface. The air outlet is located on the upper cavity wall of the detection cavity. A guide protrusion protrudes from a periphery of the air outlet. The second inclined guide surface is formed at a periphery of the guide protrusion. An inclination angle of the second inclined guide surface is greater than an inclination angle of the first inclined guide surface.

In some embodiments, the sensor assembly includes a light emitting assembly and a light receiving assembly. A mounting portion is disposed in the accommodation cavity. A receiving cavity is formed by the mounting portion and the housing. The light emitting assembly and/or the light receiving assembly are mounted in the receiving cavity. An opening is formed at a lower cavity wall of the receiving cavity, and the opening serves as the through hole.

In some embodiments, the housing includes a plurality of fins arranged at intervals in the receiving cavity along an optical axis of the light emitting assembly and/or an optical axis of the light receiving assembly. Each of the plurality of fins has a light-transmitting hole and a liquid flowing groove in communication with the light-transmitting hole, and the inclined guide surface is formed at a wall of the liquid flowing groove. In some embodiments,

In some embodiments, the light emitting assembly includes a light emitter and a transmitting lens mounted in several spaces between the plurality of fins. The receiving cavity includes a first cavity and a second cavity, the first cavity is spaced apart from the second cavity by the transmitting lens, the light emitter is mounted in the second cavity, and the opening includes a first opening formed at a lower cavity wall of the first cavity; and/or the light receiving assembly includes a light receiver and a receiving lens mounted in several spaces between the plurality of fins, the receiving cavity includes a third cavity and a fourth cavity, the third cavity is spaced apart from the fourth cavity by the receiving lens, the light receiver is mounted in the fourth cavity, and the opening includes a second opening formed at a lower cavity wall of the third cavity.

According to the oil fume sensor in the embodiments of the present disclosure, the housing has an air inlet and an air outlet that are in communication with the accommodation cavity. A light emitting assembly is configured to emit light to the accommodation cavity. A light receiving assembly is configured to receive the light emitted from the light emitting assembly. A plurality of light traps is provided in the accommodation cavity, and each of the plurality of light traps has a light trap cavity for removing light emitted into the light trap cavity.

In some embodiments, the light trap cavity has a conical cross section.

In some embodiments, an angle is formed between an optical axis of the light emitting assembly and an optical axis of the light receiving assembly. A conical vertex of the light trap cavity is located on the optical axis of the light emitting assembly, and the light trap cavities are arranged symmetrically about the optical axis of the light emitting assembly.

In some embodiments, an angle is formed between an optical axis of the light emitting assembly and an optical axis of the light receiving assembly. A conical vertex of the light trap cavity is located on the optical axis of the light receiving assembly, and the light trap cavities are arranged symmetrically about the optical axis of the light receiving assembly.

In some embodiments, a plurality of protruding ribs of an arc-shaped section protrudes from a cavity wall of the light trap cavity.

In some embodiments, roughness of a cavity wall of the light trap cavity is greater than or equal to 12.5 microns.

In some embodiments, a cavity wall of the light trap cavity is black.

In some embodiments, a region of the accommodation cavity located between the air inlet and the air outlet is provided with a detection cavity. An optical axis of the light emitting assembly and an optical axis of the light receiving assembly both pass through the detection cavity.

In some embodiments, the light emitting assembly includes a light emitter and a transmitting lens. The light emitter is located at a focal point of the transmitting lens, and light emitted from the light emitter is incident to the detection cavity by the transmitting lens.

In some embodiments, the light receiving assembly includes a light receiver and a receiving lens. The light receiver is located at a focal point of the receiving lens, and is configured to receive light by the receiving lens.

The present disclosure further provides a kitchen appliance. The kitchen appliance includes a casing, a fan assembly mounted in the casing, and the oil fume sensor according to the above embodiments. The oil fume sensor is mounted at the casing.

In some embodiments, the oil fume sensor is arranged at an air inlet of the fan assembly.

Additional aspects and advantages of the present disclosure will be in part set forth below, become apparent in part from the following description, or can be learned by practice of the present disclosure.

### BRIEF DESCRIPTION OF THE DRAWINGS

FIG. 1 is a schematic cross-sectional view of an oil fume sensor according to an embodiment of the present disclosure.
FIG. 2 is a perspective view of an oil fume sensor according to an embodiment of the present disclosure.
FIG. 3 is a schematic exploded view of an oil fume sensor according to an embodiment of the present disclosure.
FIG. 4 is another schematic cross-sectional view of an oil fume sensor according to an embodiment of the present disclosure.
FIG. 5 is a partially schematic cross-sectional view of an oil fume sensor according to an embodiment of the present disclosure.
FIG. 6 is another partially schematic cross-sectional view of an oil fume sensor according to an embodiment of the present disclosure.
FIG. 7 is an enlarged view at part A in FIG. 4.
FIG. 8 is an enlarged view at part B in FIG. 4.
FIG. 9 is an enlarged view at part C in FIG. 4.
FIG. 10 is an enlarged view at part D in FIG. 4.
FIG. 11 is yet another schematic cross-sectional view of an oil fume sensor according to an embodiment of the present disclosure.
FIG. 12 is another schematic exploded view of an oil fume sensor according to an embodiment of the present disclosure.
FIG. 13 is a schematic structural view of a bottom housing according to an embodiment of the present disclosure.
FIG. 14 is a schematic structural view of an air inlet housing according to an embodiment of the present disclosure.
FIG. 15 is another schematic cross-sectional view of an oil fume sensor according to an embodiment of the present disclosure.
FIG. 16 is yet another schematic cross-sectional view of an oil fume sensor according to an embodiment of the present disclosure.
FIG. 17 is yet another partially schematic cross-sectional view of an oil fume sensor according to an embodiment of the present disclosure.
FIG. 18 is a partially schematic structural view of a housing according to an embodiment of the present disclosure.
FIG. 19 is still yet another schematic cross-sectional view of an oil fume sensor according to an embodiment of the present disclosure.
FIG. 20 is yet another schematic cross-sectional view of an oil fume sensor according to an embodiment of the present disclosure.
FIG. 21 is a schematic principle diagram of an oil fume sensor according to an embodiment of the present disclosure.
FIG. 22 is an enlarged view at E in FIG. 21.
FIG. 23 is a schematic structural view of a kitchen appliance according to an embodiment of the present disclosure.

### Reference numerals:

oil fume sensor 100, housing 10, accommodation cavity 11, protruding portion 1101, first receiving cavity 111, second receiving cavity 112, first side wall 12, second side wall 13, exhaust cover 14, first cover body 141, receiving groove 1411, first exhaust pipe 142, sealing groove 1421, upper cover 15, second cover body 151, second exhaust pipe 152, sealing protrusion 1521, protector 153, exhaust cavity 16, bottom housing 17, first housing 171, protruding ring 1711, first air inlet pipe 172, fin 18, light-transmitting hole 181, liquid flowing groove 182, air inlet housing 19, second housing 191, second air inlet pipe 192, air inlet cavity 101, air inlet 21, air outlet 22, inclined guide surface 23, first inclined guide surface 231, second inclined guide surface 232, through hole 24, first through hole 241, second through hole 242, third through hole 243, guide protrusion 25, pressurizing channel 26, oil reduction cavity 27, detection cavity 29, sensor assembly 30, light emitting assembly 31, light emitter 311, transmitting lens 312, light receiving assembly 32, light receiver 321, receiving lens 322, light trap 33, light trap cavity 331, protruding rib 34, main control board 35, socket 351, mounting portion 40, opening 51, first opening 511, second opening 512, third opening 513, fourth opening 514, first cavity 52, second cavity 53, third cavity 54, fourth cavity 55, oil fume particle 70, kitchen appliance 1000, casing 200, fan assembly 300.

### DETAILED DESCRIPTION

The embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the accompanying drawings are illustrative only, and are intended to explain, rather than to limit the present disclosure.

In the description of the present disclosure, it is to be understood that, terms such as "center," "longitudinal," "lateral," "length," "width," "thickness," "over," "below," "front," "back," "left," "right," "vertical," "horizontal," "top," "bottom," "in," "out," "clockwise," "counterclockwise," etc., is based on the orientation or position relationship shown in the drawings, and is only for the convenience of describing the present disclosure and simplifying the description, rather than indicating or implying that the pointed device or element must have a specific orientation, or be constructed and operated in a specific orientation, and therefore cannot be understood as a limitation of the present disclosure. In addition, the terms "first" and "second" are only used for description purposes, and cannot be understood as indicating or implying relative importance or implying the number of indicated technical features. Thus, features associated with "first" and "second" may explicitly or implicitly include one or more of such features. In the description of the present disclosure, the term "plurality" means two or more, unless defined otherwise explicitly and specifically.

In the description of the present disclosure, it should be noted that, unless otherwise clearly specified and limited, terms such as "install," "connect," "connect to," and the like should be understood in a broad sense. For example, it may be a fixed connection or a detachable connection or connection as one piece; a mechanical connection or electrical connection or communication with each other; direct connection or indirect connection through an intermediate; internal communication of two components or the interaction relationship between two components. For those skilled in the art, the specific meaning of the above-mentioned terms in the present disclosure can be understood according to specific circumstances.

In the present disclosure, unless specified or limited otherwise, a structure in which a first feature is "on" or "below" a second feature may include an embodiment in which the first feature is in direct contact with the second feature, and may also include an embodiment in which the first feature and the second feature are not in direct contact with each other, but are in contact via an additional feature formed therebetween. Moreover, a first feature "on," "above," or "on top of" a second feature may include an embodiment in which the first feature is orthogonally or obliquely "on," "above," or "on top of" the second feature, or just means that the first feature is at a height higher than that of the second feature; while a first feature "below," "under," or "on bottom of" a second feature may include an embodiment in which the first feature is orthogonally or obliquely "below," "under," or "on bottom of" the second feature, or just means that the first feature is at a height lower than that of the second feature.

The following disclosure provides many different embodiments or examples for implementing different structures of the present disclosure. In order to simplify the disclosure of the present disclosure, the components and arrangements of specific examples will be described below. The components and arrangements of specific examples are only examples and are not intended to limit the present disclosure. In addition, the present disclosure may use repeated reference numerals and/or reference letters in different examples. Such repetition is for simplifying and clarifying and itself does not in indicate the relation between various embodiments and/or arrangements as discussed. In addition, the present disclosure provide examples of various specific processes and materials, but it can be appreciated by those of ordinary skill in the art that other processes can be applied and/or other materials can be used.

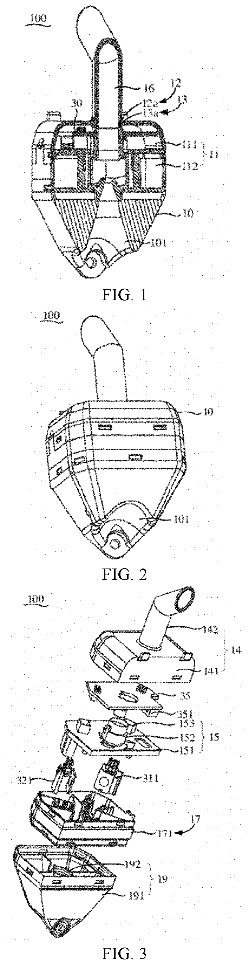

Reference is made to FIG. 1 and FIG. 2, an oil fume sensor 100 according to an embodiment of the present disclosure is provided. The oil fume sensor 100 includes a housing 10 and a sensor assembly 30. The housing 10 has an accommodation cavity 11. The accommodation cavity 11 has a first side wall 12 and a second side wall 13. The second side wall 13 is tightly fitted to an outer side of the first side wall 12 to allow the accommodation cavity 11 to be formed into an enclosed space. The sensor assembly 30 is located in the accommodation cavity 11.

According to the oil fume sensor 100 in the embodiments of the present disclosure, the second side wall 13 is tightly fitted to the outer side of the first side wall 12 to form the enclosed space, which can prevent external oil fumes from easily entering the accommodation cavity 11. Therefore, contamination of the sensor assembly 30 is further avoided or reduced, and performance and a service life of the oil fume sensor 100 are further ensured.

In some embodiments, the outer side mentioned in the embodiments of the present disclosure may be a part close to gas containing the oil fumes, i.e., a part away from the accommodation cavity 11. In contrast, the inner side mentioned in the embodiments of the present disclosure may be a part away from the gas containing the oil fumes, i.e., a part close to the accommodation cavity 11. In a case where it is difficult to determine which part is close to the gas containing the oil fumes, it may be considered that a housing 10 has the second side wall 13 should be partially sleeved over a part of a housing 10 with the first side wall 12. That is, an inner diameter of the housing 10 with the second side wall 13 should partially approximate to an outer diameter of the part of the housing 10 with the first side wall 12. Base on this, it is possible to determine the position of each of the second side wall 13 and the first side wall 12 based on this.

The external gas containing the oil fumes is difficult to enter the accommodation cavity 11 through a gap between the second side wall 13 and the first side wall 12. Even if a gap is formed between an outer side of the second side wall 13 and the outer side of the first side wall 12 due to manufacturing, usage wear, or the like, the external oil fumes-attached gas also needs to enter the accommodation cavity 11 by passing through a long gap formed between the outer side of the second side wall 13 and the outer side of the first side wall 12, which increases a probability that oil fume particles in the oil fumes-attached gas are absorbed at the outer side of the second side wall 13 or the outer side of the first side wall 12. As a result, a probability that the oil fume particles enter the accommodation cavity 11 and contaminate the sensor assembly 30 can lowered.

It should be noted that the housing 10 may include a plurality of sub-housings. Each of the plurality of sub-housings may have a first side wall 12 and a second side wall 13 fitted to each other to achieve a tight fit, or any two adjacent sub-housings of the plurality of sub-housings each have the first side wall 12 and the second side wall 13 fitted to each other. The embodiments of the present disclosure are not limited in this regard.

The tight fit between the first side wall 12 and the second side wall 13 may be achieved by injecting an adhesive between the second side wall 13 and the first side wall 12, and may also be achieved through an interference fit between the second side wall 13 and the first side wall 12, and the present disclosure is not limited in this regard.

The sensor assembly 100 may be a gas component detection device. As an example, the sensor assembly 100 is applied in a kitchen appliance1000. When a user uses the kitchen appliance 1000 to generate the oil fumes, predetermined organic molecules may be generated. Generally, a concentration of the organic molecules is proportional to a concentration of the oil fumes. Therefore, whether the user is cooking and the concentration of the oil fumes may be determined by detecting the current organic molecules and the concentration of the organic molecules. In some embodiments, the gas component detection device may employ a volatile organic compounds (VOC) sensor.

The oil fume sensor 100 may be a device for detecting a gas particle concentration. Moreover, oil fume particles generated by the user using the kitchen appliance 1000 may be mixed in a gas and enter the oil fume sensor 100 together. Therefore, it can be determined that whether the user is cooking by detecting the gas particle concentration. A wide variety of device for detecting the gas particle concentration is available, which may be an infrared detection device, a laser detection device, or the like, and the present disclosure is not limited in this regard.

As an example, the oil fume sensor 100 is the infrared detection device. The sensor assembly 30 includes, but is not limited to, a light emitter 311, a light receiver 321, and a main control board 35. The light emitter 311 is configured to emit light to a gas possibly attached with the oil fumes. The light receiver 321 is configured to receive the light emitted from the light emitter 311. The main control board 35 is electrically connected to the light emitter 311 and the light receiver 321. Therefore, the light emitter 311 may be controlled to emit light. Moreover, a current oil fume situation attached to the gas is determined based on light information received by the light receiver 321. It can be understood that a particle size of the oil fume particles is in a range of 100 nm to 10 µm. When the oil fume particles pass through a light path of the light emitter 311, shielding, scattering, and diffraction of infrared light may occur. That is, the oil fume particles may affect an intensity of the light received by the light receiver 321, thereby changing the light information obtained by the main control board 35.

The main control board 35 may also be provided with a communication module. The communication module may be connected to a mobile terminal such as a mobile phone, a tablet, and a computer, which is convenient for the user to control the oil fume sensor 100 to operate. Moreover, the communication module may also be electrically connected to or in a communication connection with other components of the kitchen appliance 1000, to facilitate opening or closing the oil fume sensor 100 based on a state of a switch of the kitchen appliance 1000, and opening and closing a motor or increasing and reducing power of the motor based on a detection result of the oil fume sensor 100.

Referring to FIG. 1, in some embodiments, the second side wall 13 is in an interference fit with the outer side of the first side wall 12 to allow for a tight fit between the second side wall 13 and the first side wall 12. After the second side wall 13 and the outer side of the first side wall 12 are mounted, the tight fit between the second side wall 13 and the outer side of the first side wall 12 may be achieved without requiring other steps. Therefore, operation steps are simplified, and manufacturing cost is lowered.

In some embodiments, in order to facilitate the mounting of the second side wall 13 and the first side wall 12, inclined guide surfaces may be formed at the outer side of the first side wall 12, and may also be located at the second side wall 13. Moreover, the inclined guide surfaces may be fitted to each other and also be located on each of the first side wall 12 and the second side wall 13, to guide the mounting of the second side wall 13 and the first side wall 12. After the mounting is completed, the interference fit between the second side wall 13 and the first side wall 12 is formed.

In some embodiments, referring to FIG. 1 and FIG. 3, the sensor assembly 30 includes a main control board 35. The accommodation cavity 11 includes a first receiving cavity 111. The main control board 35 is accommodated in the first receiving cavity 111. The housing 10 includes an exhaust cover 14 and an upper cover 15. The exhaust cover 14 includes a first cover body 141 and a first exhaust pipe 142 penetrating the first cover body 141. The first exhaust pipe 142 has a sealing groove 1421 formed at an end of the first exhaust pipe 142. The upper cover 15 includes a second cover body 151 and a second exhaust pipe 152 penetrating the second cover body 151. The second exhaust pipe 152 is provided with a sealing protrusion 1521 engaged in the sealing groove 1421. An interior of the first exhaust pipe 142 is in communication with an interior of the second exhaust pipe 152 to form an exhaust cavity 16, and the first receiving cavity 111 is formed by the first cover body 141, the second cover body 151, the first exhaust pipe 142, and the second exhaust pipe 152.

The first receiving cavity 111 for accommodating the main control board 35 is formed by the exhaust cover 14 and the upper cover 15 together. In this way, it is possible to protect the main control board 35 from being in contact with a gas containing the oil fumes. Therefore, a probability of failure of the main control board 35 is lowered, and a service life of the main control board 35 is prolonged.

In some embodiments, the first cover body 141 and the first exhaust pipe 142 may be of an integrated structure. For example, the first cover body 141 and the first exhaust pipe 142 are formed through injection molding. For another example, the first cover body 141 and the first exhaust pipe 142 are formed by cutting simultaneously. In this way, manufacturing of each of the first cover body 141 and the first exhaust pipe 142 may be simple. Moreover, no connection gap is formed between the first cover body 141 and the first exhaust pipe 142, which increases airtightness of the first receiving cavity 111.

The first cover body 141 is of various shapes. For example, the first cover body 141 may be in the shape of a cuboid, a hemisphere, or the like, and the present disclosure is not limited in this regard.

The first exhaust pipe 142 may extend in a vertical direction, in a horizontal direction, or in a direction at any angle relative to the vertical direction. In an embodiment, the first exhaust pipe 142 extends in the vertical direction. An oil drop condensed in the first exhaust pipe 142 may drip under the gravity, to reduce a speed at which the first exhaust pipe 142 is contaminated by an oil stain, and increase a service life of the first exhaust pipe 142. The first exhaust pipe 142 has various types, and a cross section of the first exhaust pipe 142 may be square, circular, or the like, and the present disclosure is not limited in this regard.

The second cover body 151 and the second exhaust pipe 152 may be of an integrated structure. For example, the second cover body 151 and the second exhaust pipe 152 are integrally formed through injection molding. For another example, the second cover body 151 and the second exhaust pipe 152 are formed by cutting simultaneously. In this way, the second cover body 151 and the second exhaust pipe 152 may be produced simply. Moreover, no connection gap is formed between the second cover body 151 and the second exhaust pipe 152, which increases the airtightness of the first receiving cavity 111.

An extending direction of the second exhaust pipe 152 may be the same as an extending direction of the first exhaust pipe 142. In this way, the gas adhered with the oil fumes may move in a same direction in the exhaust cavity 16 formed by the second exhaust pipe 152 and the first exhaust pipe 142. Therefore, a probability that the oil fume particles are maintained on the first exhaust pipe 142 and the second exhaust pipe 152 is decreased, and a speed at which the oil fume sensor 100 is contaminated by the oil fumes is slowed.

There are various arrangements of the first side wall 12 and the second side wall 13 at each of the sealing groove 1421 and the sealing protrusion 1521, and the detailed description thereof will be set forth below.

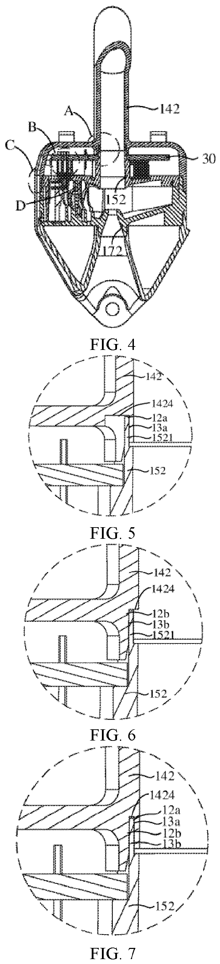

In some embodiments, referring to FIG. 4 and FIG. 5, the first side wall 12a is formed at an outer side wall of the sealing protrusion 1521, and the second side wall 13a is formed at an inner side wall of the sealing groove 1421.

With this arrangement, the first side wall 12a of the sealing protrusion 1521 is tightly fitted to the second side wall 13a of the sealing groove 1421. Thus, it is possible to effectively increase the airtightness of the first receiving cavity 111, and reduces a probability that the oil fumes enter the first receiving cavity 111 from a connection between the first exhaust pipe 142 and the second exhaust pipe 152. In some embodiments, a tight fit between the second side wall 13a of the sealing groove 1421 and the outer side wall of the sealing protrusion 1521 may be realized through an interference fit between the second side wall 13a of the sealing groove 1421 and the outer side wall of the sealing protrusion 1521.

It should be noted that the outer side wall of the sealing protrusion 1521 refers to a wall of a side of the sealing protrusion 1521 away from the accommodation cavity 11, and the inner side wall of the sealing groove 1421 refers to a wall of a side of the sealing groove 1421 close to the accommodation cavity 11. In some embodiments, referring to FIG. 6, the second side wall 13b is formed at an inner side wall of the sealing protrusion 1521, and the first side wall 12b is formed at an outer side wall of the sealing groove 1421. The second side wall 13b of the sealing protrusion 1521 is tightly fitted to the first side wall 12b of the sealing groove 1421. Therefore, it is possible to effectually increase the airtightness of the first receiving cavity 111, and reduces the probability that the oil fumes enter the first receiving cavity 111 from the connection between the first exhaust pipe 142 and the second exhaust pipe 152.

In some embodiments, referring to FIG. 7, the first side wall 12a is formed at the outer side wall of the sealing protrusion 1521, and the second side wall 13b is formed at the inner side wall of the sealing protrusion 1521. The second side wall 13a is formed at the inner side wall of the sealing groove 1421, and the first side wall 12b is formed at the outer side wall of the sealing groove 1421.

With this arrangement, the airtightness of the first receiving cavity 111 can be ensured. In addition, even if the tight fit between the first side wall 12and the second side wall 13 fails due to manufacturing, wear, and other reasons, the oil fumes still needs to pass through a gap formed between the second side wall 13a of the sealing groove 1421 and the first side wall 12a of the sealing protrusion 1521, then pass through a gap formed between the first side wall 12b of the sealing groove 1421 and the second side wall 13b of the sealing protrusion 1521, and enter the first receiving cavity 111, to effectively isolate the first receiving cavity 111 from the oil fumes.

In conclusion, the sealing protrusion 1521 may has a first side wall 12a, and the sealing groove 1421 has a corresponding second side wall 13a. The sealing protrusion 1521 may also have a second side wall 13b, and the sealing groove 1421 may have a corresponding first side wall 12b. The sealing protrusion 1521 may further have the first side wall 12a and the second side wall 13b, and the sealing groove 1421 may have a corresponding second side wall 13a and a corresponding first side wall 12b.

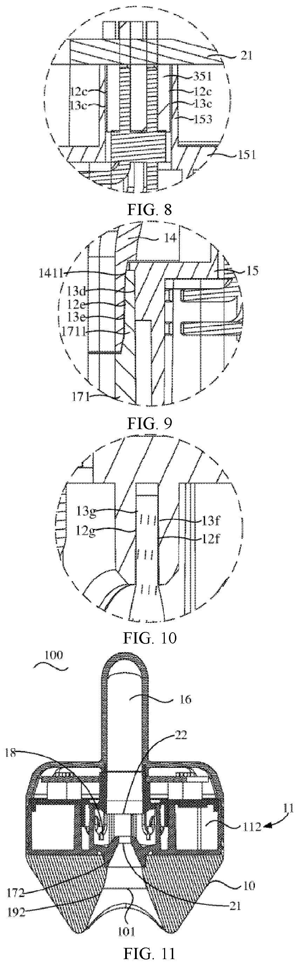

In some embodiments, referring to FIG. 4 and FIG. 8, the upper cover 15 is provided with a hollow protector 153 protruding from the upper cover 15. The second side wall 13c is formed at an inner edge of the protector 153. The main control board 35 is provided with a socket 351. The socket 351 protrudes from the main control board 35. The first side wall 12c is formed at a periphery of the socket 351.

The second side wall 13c of the protector 153 is fitted to the first side wall 12c of the socket 351, which efficaciously prevents the oil fumes from entering the first receiving cavity 111 from a gap between the socket 351 and the protector 153.

In some embodiments, the sensor assembly 30 may include the light emitter 311 and the light receiver 321. The light emitter 311 and the light receiver 321 may be provided with a inserter in plug-fit between an inserter and the socket 351 to realize a connection with the main control board 35, thereby receiving control of the main control board 35 and transmitting information to the main control board 35. However, in a case where the light emitter 311 and the light receiver 321 are not located in the first receiving cavity 111, an engagement between the inserter and the socket 351 may affect the airtightness of the first receiving cavity 111. Therefore, the protector 153 is provided at the upper cover 15 to be tightly fitted to the socket 351, and the inserter is inserted to the socket 351, which can ensure the airtightness of the first receiving cavity 111.

In some embodiments, referring to FIG. 1 and FIG. 3, the sensor assembly 30 includes a light emitter 311, a light receiver 321, and a lens. The accommodation cavity 11 includes a second receiving cavity 112. The light emitter 331 and the light receiver 321 are accommodated in the second receiving cavity 112. The housing 10 includes an upper cover 15 and a bottom housing 17. The bottom housing 17 includes a first housing 171 and a first air inlet pipe 172 penetrating the first housing 171. The second receiving cavity 112 is formed by the upper cover 15, the first housing 171, and the lens. The upper cover 15 and the bottom housing 17 together form the second receiving cavity 112 for accommodating the light emitter 311 and the light receiver 321, thereby protecting the light emitter 311 and the light receiver 321 from being in contact with the gas containing the oil fumes. Therefore, it is possible to lower a probability of failures of the light emitter 311 and the light receiver 321. Moreover, a service life of the light emitter 311 and a service life of the light receiver 321 are prolonged.

In some embodiments, the first housing 171 and the first air inlet pipe 172 may be of an integrated structure. For example, the first housing 17 and the first air inlet pipe 172 are integrally formed through injection molding. For another example, the first housing 171 and the first air inlet pipe 172 are formed through simultaneous cutting. Therefore, manufacturing of each of the first housing 171 and the first air inlet pipe 172 may be simple. Moreover, no connection gap is formed between the first housing 171 and the first air inlet pipe 172, which improves airtightness of the second receiving cavity 112.

The first air inlet pipe 172 may extend in the vertical direction, in the horizontal direction, or in the direction at any angle relative to the vertical direction. In an embodiment, the first air inlet pipe 172 extends in the vertical direction. The oil drop condensed in the first air inlet pipe 172 may drip under the gravity, to reduce the speed at which the first air inlet pipe 172 is contaminated by the oil stains and prolong service life of the first air inlet pipe 172. The first air inlet pipe 172 may be in various shapes, and its cross section may be square, circular, or the like, and the present disclosure is not limited in this regard.

Further, referring to FIG. 4 and FIG. 9, the first side wall 12d is formed at a peripheral wall of the upper cover 15. The first housing 10 is provided with a protruding ring 1711. The second side wall 13d is formed at an inner side wall of the protruding ring 1711. The first side wall 12d of the upper cover 15 is tightly fitted to the second side wall 13d of the protruding ring 1711, which effectively increases the airtightness of the second receiving cavity 112 and reduce a probability that the oil fumes enter the second receiving cavity 112 from a connection between the upper cover 15 and the first housing 171. In some embodiments, the first side wall 12d of the upper cover 15 is in interference fit with the second side wall 13d of the protruding ring 1711 to allow for a tight fit between the first side wall 12d of the upper cover 15 and the second side wall 13d of the protruding ring 1711. In this way, the first side wall 12d of the upper cover 15 and the second side wall 13d of the protruding ring 1711 may each have inclined guide surfaces fitted to each other, allowing the first side wall 12d of the upper cover 15 to be in clearance fit or a transition fit with the second side wall 13d of the protruding ring 1711 during assembly. Moreover, the interference fit between the first side wall 12d of the upper cover 15 and the second side wall 13d of the protruding ring 1711 is formed after the assembly is completed, to facilitate the assembly of the upper cover 15 and the first housing 171.

In some embodiments, the housing 10 further includes an exhaust cover 14. The exhaust cover 14 has a receiving groove 1411. The second side wall 13e is formed at an inner side wall of the receiving groove 1411. The first housing 171 is provided with a protruding ring 1711. The first side wall 12e is formed at an outer side wall of the protruding ring 1711.

The first side wall 12e of the protruding ring 1711 is tightly fitted to the second side wall 13e of the receiving groove 1411, which lowers a probability that the oil fumes enter the second receiving cavity 112 from a connection between the first housing 171 and the exhaust cover 14.

In some embodiments, a direction in which the first side wall 12e of the protruding ring 1711 is assembled with the second side wall 13 of the receiving groove 1411 refers to as an assembly direction. A snap protrudes from the first housing 171 in a direction perpendicular to the assembly direction. The exhaust cover 14 may have a through hole, into which the snap is engaged. In this way, after the first side wall 12e of the protruding ring 1711 and the second side wall 13e of the receiving groove 1411 are assembled, the snap is in snap fit with the through hole to restrict a position of the bottom housing 17 and a position of the exhaust cover 14. As a result, it is possible to ensure a constant relative position relationship between the bottom housing 17 and the exhaust cover 14.

Further, the first side wall 12e and the second side wall 13d may be formed at each of two sides of the protruding ring 1711. The second side wall 13d at an inner side of the protruding ring 1711 is fitted to the first side wall 12d of the upper cover 15. The first side wall 12e at the outer side of the protruding ring 1711 is engaged in the receiving groove 1411 of the exhaust cover 14. A limiting protrusion may protrude from the upper cover 15. The limiting protrusion is located in the receiving groove 1411 and abuts against the protruding ring 1711. In this way, the limiting protrusion is sandwiched between a wall of the receiving groove 1411 and the protruding ring 1711, to restrict a position of the upper cover 15. After the snap of the first housing 171 is engaged in the through hole of the exhaust cover 14, a relative position relationship between the upper cover 15 and the first housing 171 and a relative position relationship between the upper cover 15 and the exhaust cover 14 can be maintained.

In some embodiments, the housing 10 further includes a fixing portion configured to restrict a position of the lens. The first side wall 12f is formed at an outer side wall of the lens, and the second side wall 13f is formed at an inner side wall of the fixing portion; and/or the second side wall 13g is formed at an inner side wall of the lens, and the first side wall 12g is formed at an outer side wall of the fixing portion. The fixing portion is tightly fitted to the lens. Therefore, it is possible to reduce a probability that the oil fumes enter the second receiving cavity 112 from a connection between the fixing portion and the lens.

In some embodiments, the fixing portion may include a fin. In an embodiment, the fin is located at an outer side of the lens. The second sidewall 13f is formed at the fin, and the first sidewall 12f is formed at the lens.

In an embodiment, the fin is located on the inner side of the lens. The first side wall 12g is formed at the fin, and the second side wall 13g is formed at the lens.

In an embodiment, the fixing portion includes at least two fins. The lens is located in a space between two adjacent fins. In this way, the second side wall 13f is formed at an inner side wall of a fin of two adjacent fins close to an outer side. The first side wall 12f is formed at the outer side of the lens. The first side wall 12g is formed at an outer side wall of a fin of the two adjacent fins close to the inner side. The second side wall 13g is formed at the inner side wall of the lens. It should be noted that a position close to the gas containing the oil fumes is referred to as the outer side, and a position away from the gas containing the oil fumes is referred to as the inner side.

In some embodiments, an end of the fin close to the lens has a semicircular through hole. The fixing portion includes a plurality of fins. The plurality of fins is mounted at the first housing 171 and the second cover body 151. The first housing 171 and the second cover body 151 are assembled together to form the second receiving cavity 112. An upper fin and a lower fin are assembled together to form a circular through hole. In this way, light to the lens and light from the light emitter 311 or light to the light receiver 321 may be shaped. In addition, when during the using of the fins, the two fins 18 are assembled together to form a circular aperture. During manufacturing, the fins with the semicircular through holes are produced respectively, to achieve convenient de-molding.

In some embodiments, referring to FIG. 1 to FIG. 4, in a further embodiment, the housing 10 includes an exhaust cover 14, an upper cover 15, a bottom housing 17, and an air inlet housing 19. The air inlet housing 19 includes a second housing 191 and a second air inlet pipe 192 penetrating the second housing 191. The bottom housing 17 is provided with the first air inlet pipe 172. An interior of the first air inlet pipe 172 is in communication with an interior of the second air inlet pipe 192 to form an air inlet cavity 101. The exhaust cover 14 is provided with a first exhaust pipe 142. The upper cover 15 is provided with a second exhaust pipe 152. An interior of the first exhaust pipe 142 is in communication with an interior of the second exhaust pipe 152 to form the exhaust cavity 16. The housing 10 further includes a detection cavity located between the air inlet cavity 101 and the exhaust cavity 16, to allow the sensor assembly 30 to detect gas in the detection cavity. The sensor assembly 30 includes the main control board 35, the light emitter 311, the light receiver 321, and the lens. Each of the light emitter 311 and the light receiver 321 is provided with a lens. The gas containing the oil fumes enters the detection cavity from the air inlet cavity 101. Light is emitted from the light emitter 311 and enters the detection cavity through the lens of the light emitter 311. The gas in the detection cavity affects the light emitted from the light emitter 311. The light receiver 321 can receive the light affected by the gas in the detection cavity through the lens of the light receiver 321, and transmits the received information to the main control board 35. The main control board 35 can analyze a concentration of the oil fume particles in the gas based on the influence.

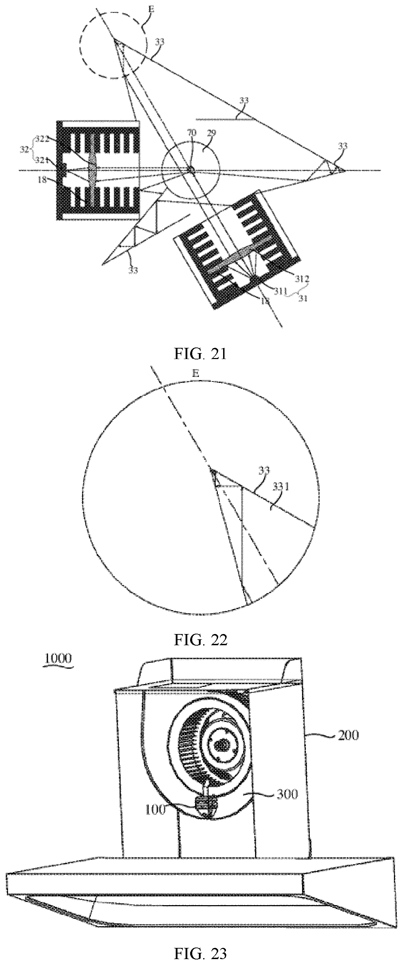

Referring to FIG. 23, an embodiment of the present disclosure provides a kitchen appliance 1000. The kitchen appliance 1000 includes a casing 200, a fan assembly 300, and an oil fume sensor 100. The fan assembly 300 is mounted in the casing 200. According to the kitchen appliance 1000 in the embodiments of the present disclosure, the second side wall 13 is tightly fitted to the outer side of the first side wall 12 to form the enclosed space. As a result, it is impossible for external oil fumes to easily enter the accommodation cavity 11. In this way, pollution of the sensor assembly 30 is further avoided or reduced, and performance and a service life of the oil fume sensor 100 are ensured.

In some embodiments, the kitchen appliance 1000 in the embodiments of the present disclosure includes, but is not limited to, a deflector assembly and a check valve. The casing 200 is arranged at the deflector assembly. The deflector assembly has a smoke collecting cavity and a plurality of function buttons. An oil mesh and a top plate are provided in the smoke collecting cavity, and the plurality of function buttons may be used for the user to input an operation instruction. The fan assembly 300 is provided in the casing 200. The fan assembly 300 has a volute, a fan, an air inlet, and an air outlet. The fan is arranged in the volute. A volute air duct is formed in the volute. The air inlet is configured to allow the oil fumes to enter the fan assembly 300. The air outlet is in communication with the volute air duct and is configured to allow the oil fumes to be discharged out of the fan assembly 300. The check valve is connected to the top of the casing 200. The check valve internally has a check valve air duct. It can be understood that the check valve refers to a valve that an openable/closable member is a circular valve clack and is configured to block backflow of a medium by means of its own weight and a pressure of the medium. The check valve may be a lifting check valve and a swing check valve.

The oil fume sensor 100 is arranged at a position on the kitchen appliance 1000 through which the gas containing the oil fumes passes, such as a center of a deflector and an air inlet of the fan assembly 200. In this way, the gas containing the oil fumes may be allowed for entering the oil fume sensor 100 for detection.

In some embodiments, after the oil fumes are generated around the kitchen appliance 1000, the oil fumes move under the action of a household appliance 1000 and enter the air inlet cavity 101 of the oil fume sensor 100 arranged in the casing 200. The housing 10 further includes a detection cavity. The detection cavity is located between the air inlet cavity 101 and the exhaust cavity 16, allowing the sensor assembly 30 to detect the gas in the detection cavity. The sensor assembly 30 includes the main control board 35, the light emitter 311, the light receiver 321, and the lens. Each of the light emitter 311 and the light receiver 321 is provided with a lens. The gas containing the oil fumes enters the detection cavity from the air inlet cavity 101. Light is emitted from the light emitter 311 and enter the detection cavity through the lens of the light emitter 311. The gas in the detection cavity will affect the light emitted from the light emitter 311. The light receiver 321 can receive the light affected by the gas in the detection cavity through the lens of the light receiver 321, and transmits the received information to the main control board 35. The main control board 35 can analyze the concentration of the oil fume particles in the gas on the basis of the influence. The detected gas is discharged out of the oil fume sensor 100 through the exhaust cavity 16.

In some embodiments, the exhaust cavity 16 of the oil fume sensor 100 faces towards the air inlet of the fan assembly 300, and the air inlet cavity 101 of the oil fume sensor 100 is spaced apart from the air inlet of the fan assembly 300. In this way, the exhaust cavity 16 and the air inlet cavity 101 of the oil fume sensor 100 generate a pressure difference after the fan assembly 300 operates, allowing the airflow containing the oil fumes to flow into the air inlet cavity under the pressure difference. In some embodiments, the air inlet cavity 101 of the oil fume sensor 100 is spaced apart from the air inlet of the fan assembly 300. However, under the action of the fan assembly 300, compared with an atmospheric pressure, a predetermined negative pressure such as -SPa, may be generated between the air inlet cavity 101 of the oil fume sensor 100 and the air inlet of the fan assembly 300. By the fan assembly 300, a negative pressure of about -30Pa may be generated in the exhaust cavity 16 of the oil fume sensor 100 relative to the atmospheric pressure. Therefore, a pressure difference of approximately 25Pa is formed between the air inlet cavity 101 and the exhaust cavity 16, to drive the gas containing the oil fumes to move.

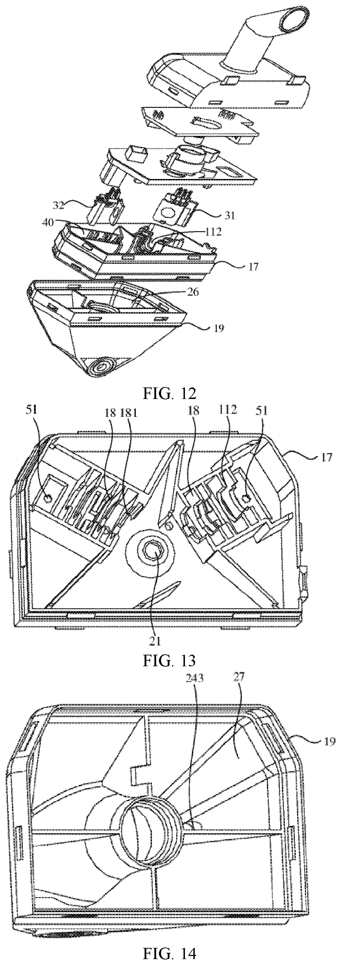

Referring to FIG. 11 and FIG. 12, an embodiment of the present disclosure provides an oil fume sensor. The oil fume sensor includes a housing 10 and a sensor assembly 30. The housing 10 has an accommodation cavity 11, an air inlet 21, and an air outlet 22. The air inlet 21 and the air outlet 22 are each in communication with the accommodation cavity 11. The housing 10 includes a lower housing and an air inlet pipe penetrating the lower housing. The air inlet pipe internally has an air inlet cavity 101. The air inlet 21 is formed at a position communicating between the air inlet cavity 101 and the accommodation cavity 11, and the air inlet cavity 101 is tapered towards the air inlet 21. The sensor assembly 30 is located in the accommodation cavity 11.

In the oil fume sensor according to the embodiments of the present disclosure, the air inlet cavity 101 is tapered, allowing a speed at which the oil fumes enters the accommodation cavity 11 from the air inlet cavity 101 to be constantly accelerated as the air inlet cavity 101 is tapered. In this way, the oil fumes are accelerated to pass through the accommodation cavity 11 and quickly discharged from the air outlet 22. Therefore, it is possible to reduce dissipation of the oil fumes in the accommodation cavity 11. Therefore, a probability that the oil fumes are condensed at the sensor assembly 30 is lowered, and the service life and performance of the oil fume sensor are enhanced.

In some embodiments, the housing 10 is in various shapes, which may have cuboid, spheroidal, pyramidal, or other shapes, and the present disclosure is not limited in this regard. In an embodiment, the housing 10 is of a shuttle shape. That is, an outer diameter of the housing 10 gradually increases from top to bottom and then gradually decreases from top to bottom. In this way, an oil drop condensed at an outer wall of the housing 10 may naturally drip under the gravity. Moreover, a speed at which the outer wall of the housing 10 is contaminated by the oil stains is reduced.

The accommodation cavity 11 may be in various shapes, such as a cuboid, spheroidal, pyramidal shape, or other shapes. The shape of the accommodation cavity 11 may follow the shape of the housing 10, and the present disclosure is not limited in this regard. The lower housing and the air inlet pipe may be integrally formed through injection molding. For example, the lower housing and the air inlet pipe are formed through simultaneous cutting. In this way, manufacturing of each of the lower housing and the air inlet pipe is simple. The lower housing has various shapes, which may be cuboid, semispherical, or other shape, and the present disclosure is not limited in this regard. The air inlet pipe may extend in the vertical direction, in the horizontal direction, or in the direction at any angle relative to the vertical direction. In an embodiment, the air inlet pipe extends in the vertical direction, thereby allowing the oil drop condensed at the air inlet pipe to drip under the gravity. Therefore, a speed at which the air inlet pipe is contaminated by the oil stains is lowered. Moreover, a service life of the air inlet pipe is prolonged. The air inlet pipe has various shapes, and thus has a cross section of a square, circular shape, or other shape, and the present disclosure is not limited in this regard.

The air inlet cavity 101 is tapered towards the air inlet 21. In some embodiments, it can be understood that a cross-sectional area of the air inlet cavity 101 gradually decreases towards the air inlet 21. In this case, the cross section of the air inlet cavity 101 is vertically close to or away from the air inlet 21.

It should be noted that the air inlet cavity 101 may have a constant reduction speed for the cross-sectional area. That is, the cross-sectional area of the air inlet cavity 101 is in direct proportion to a distance between the air inlet cavity 101 and the air inlet 21. The reduction speed of the cross-sectional area of the air inlet cavity 101 may also increase and then decrease. The reduction speed of the cross-sectional area of the air inlet cavity 101 may also decrease and then increase; or the cross-sectional area of the air inlet cavity 101 may also decrease, then unchanged, and then decrease. The embodiments of the present disclosure do not enumerate this herein.

It should be noted that even if a cavity wall of the air inlet cavity 101 partially and gradually increases towards the air inlet 21. As long as the air inlet cavity 101 appears to be tapered as a whole, it can be considered that the air inlet cavity 101 is tapered towards the air inlet 21.

The air inlet 21 is configured to allow for an inflow of airflow in the air inlet cavity 101 into the accommodation cavity 11. The air outlet 22 is configured to allow for an outflow of airflow in the accommodation cavity 11 from the accommodation cavity 11. Various relative position relationships is existed between the air inlet 21 and the air outlet 22. For example, the air inlet 21 and the air outlet 22 may be located at left and right ends of the accommodation cavity 11, and the air inlet 21 is arranged at a same level as the air outlet 22. For example, the air inlet 21 and the air outlet 22 are located at an end of the accommodation cavity 11, and the air inlet 21 is located below the air outlet 22. The embodiments of the present disclosure do not enumerate this herein.

In some embodiments, the air inlet 21 and the air outlet 22 may be located at two ends of the accommodation cavity 11. Moreover, oil fume particles discharged through the air inlet 21 into the accommodation cavity 11 move away from the air inlet 21. In this way, the oil fume particles can move towards the air outlet 22 while moving away from the air inlet 21. In this way, it is possible to increase a probability that the oil fume particles are discharged through the air outlet 22 at a faster speed after entering the accommodation cavity 11 from the air inlet 21. As a result, a probability that the oil fume particles escape in the accommodation cavity 11 and contaminate the accommodation cavity 11 can be lowered. The air inlet 21 and the air outlet 22 may be located at an upper end and a lower end of the accommodation cavity 11, and the air inlet 21 is located directly below the air outlet 22. Since the oil fume particles generally move upwards. In this way, the oil fume particles entering the accommodation cavity 11 from the air inlet 21 can naturally move to the air outlet 22 to be discharged. Moreover, the probability that the oil fume particles escape in the accommodation cavity 11 is further lowered. In addition, movement positions of the oil fume particles are concentrated, which is more conducive to detection for the oil fume particles by the sensor assembly 30.

The oil fume sensor 100 may be an infrared detection sensor, a laser detection sensor, or the like, and the present disclosure is not limited in this regard. A detailed description will be provided by taking the infrared detection sensor as the oil fume sensor 100.

The sensor assembly 30 may include a light emitting assembly 31 and a light receiving assembly 32. The light emitting assembly 31 is configured to emit light to the accommodation cavity 11. The light receiving assembly 32 is configured to receive the light emitted from the light emitting assembly 31, and may also output an electrical signal based on the received light. Generally, the particle size of the oil fume particles is in the range of 100nm to 10µm. In an embodiment, when the oil fume particles pass through a light path of the infrared light emitted from the light emitting assembly 31, the infrared light may be shielded, scattered, and diffracted, i.e., the oil fumes particles of the accommodation cavity 11 will affect an intensity at which the light receiving assembly 32 receives the light emitted from the light emitting assembly 31 to change the light received by the light receiving assembly 32, thereby determining the concentration of the oil fumes particles based on this change.

In some embodiments, the sensor assembly 30 further includes the main control board. The light receiving assembly 32 may be connected to the main control board and output the electrical signal to the main control board based on the received light. The main control board may also be provided with the communication module. The communication module may be connected to a mobile terminal such as a mobile phone, a tablet, and a computer, which is convenient for the user to control the oil fume sensor to operate. Moreover, the communication module may also be electrically connected to or in a communication connection with other components of the kitchen appliance 1000, to facilitate opening or closing the oil fume sensor based on the state of the switch of the kitchen appliance 1000, and opening and closing a fan assembly or increasing and reducing power of the fan assembly based on a detection result of the oil fume sensor. In an example, a correspondence between an oil fume and a fan air volume may be established by simulating a scenario in which the kitchen appliance is actually used, and the fume concentration may be calibrated by the electrical signal output by the light receiving assembly 32. The corresponding air volume is achieved through a rotational speed of the fan assembly, which can improve an oil fume suction effect.

In some embodiments, referring to FIG. 11, the air inlet cavity 101 is in a truncated cone shape. The oil fumes can enter the accommodation cavity 11 along a flat cavity wall of the air inlet cavity 101. Thus, it is possible to reduce a possibility that the oil fume is adhered to the cavity wall of the air inlet cavity 101. Therefore, a detection error of the oil fumes is reduced, and the service life of the air inlet cavity 101 is prolonged. In addition, the cavity wall of the air inlet cavity 101 is smooth, which also facilitates an oil drop condensed in the air inlet cavity 101 sliding off from the cavity wall of the air inlet cavity 101, and lowers a probability that the oil drop is adhered to the air inlet cavity 101.

In some embodiments, referring to FIG. 11 and FIG. 12, the sensor assembly 30 includes a light emitting assembly 31 and a light receiving assembly 32. A mounting portion 40 is provided in the accommodation cavity 11. A second receiving cavity 112 is formed by the mounting portion 40 and the housing 10. The light emitting assembly 31 and/or the light receiving assembly 32 are mounted in the second receiving cavity 112. The housing 10 has a pressurizing channel 26. One end of the pressurizing channel 26 is in communication with the second receiving cavity 112, and the other end of the pressurizing channel 26 is in communication with an external environment.

When flowing, an external airflow partially enters the second receiving cavity 112. In this way, an air pressure in the second receiving cavity 112 is increased, and there is a constant air pressure at a position communicating between the accommodation cavity 11 and each of the air inlet 21 and the air outlet 22. Therefore, an air pressure difference is generated between the second receiving cavity 112 and other parts of the accommodation cavity 11, which can prevent oil fumes at the communication between the accommodation cavity 11 and each of the air inlet 21 and the air outlet 22 from entering the second receiving cavity 112. In addition, once an oil drop is condensed in the second receiving cavity 112, the oil drop may also be discharged to the external environment through the pressurizing channel 26.

In some embodiments, the second receiving cavity 112, in addition to being in communication with the pressurizing channel 26, should be in a closed state, to allow for an inflow of the airflow into the second receiving cavity 112 from the pressurizing channel 26 without an outflow from other parts. In this way, it is possible to ensure that the second receiving cavity 112 has a high air pressure. It should be noted that the end of the pressurizing channel 26 in communication with the external environment should be located in an opposite direction to an airflow direction of the household appliance 1000. Thus, when the airflow flows under the driving of the household appliance 1000, kinetic energy of the airflow can drive the airflow to enter the pressurizing channel 26 and the second receiving cavity 112, and a dynamic pressure of the airflow allows an increase in a static pressure in the second receiving cavity 112. The oil fumes is input into the accommodation cavity 11 through the air inlet 21, and the oil fumes in the accommodation cavity 11 is discharged through the air outlet 22. Therefore, although the inflow of airflow occurs in the accommodation cavity 11, the pressure in the accommodation cavity 11 cannot be increased. As a result, a pressure difference between the accommodation cavity 11 and the second receiving cavity 112. The oil fumes at the communication between the accommodation cavity 11 and each of the air inlet 21 and the air outlet 22 are difficult to enter the second receiving cavity 112 due to the pressure difference, which can prevent the oil fumes entering the accommodation cavity 11 through the air inlet 21 from escaping to the second receiving cavity 112.

The mounting portion 40 may be formed in several manners. The mounting portion 40 may be formed by assembling a plurality of mounting plates, or formed by turning on the material, and the present is not limited in this regard.

Further, referring to FIG. 11 to FIG. 14, the lower housing includes a bottom housing 17 and an air inlet housing 19. The second receiving cavity 112 is formed by the bottom housing 17 and the mounting portion 40. An air inlet pipe includes a first air inlet pipe 172 and a second air inlet pipe 192. The first air inlet pipe 172 penetrates the bottom housing 17. The second air inlet pipe 192 penetrates the air inlet housing 19. An interior of the first air inlet pipe 172 is in communication with an interior of the second air inlet pipe 192 to form the air inlet cavity 101.An opening 51 is formed at the bottom housing 17, and a third through hole 243 is formed at the air inlet housing 19. The opening 51 is in communication with the third through hole 243 to form the pressurizing channel 26.

The bottom housing 17 and the air inlet housing 19 may be respectively manufactured, which can flexibly adjust manufacturing steps as desired. In addition, the sensor assembly 30, the mounting portion 40, and the like may be assembled with the air inlet housing 19 after being assembled with the bottom housing 17. That is, the manufacturing steps may be flexibly adjusted as desired. Moreover, it is convenient to replace the bottom housing 17 or the air inlet housing 19 when the bottom housing 17 or the air inlet housing 19 is partially damaged, rather than replacing the whole lower housing.

In some embodiments, the bottom housing 17 and the first air inlet pipe 172 may be integrally formed through injection molding. For example, the bottom housing 17 and the first air inlet pipe 172 are formed through simultaneous cutting. As a result, the manufacturing of the lower housing and the air inlet pipe is simple. The bottom housing 17 and the first air inlet pipe 172 may also be separately arranged. For example, the bottom housing 17 is removably connected to the first air inlet pipe 172 by threads, bolts, or through snap-fit. For another example, the bottom housing 17 is fixedly connected to the first air inlet pipe 172 through bonding, welding, or the like. Therefore, the manufacturing of each of the bottom housing 17 and the first air inlet pipe 172 may be conveniently adjusted as desired.

The air inlet housing 19 and the second air inlet pipe 192 may be integrally formed through injection molding. For example, the air inlet housing 19 and the second air inlet pipe 192 are formed through simultaneous cutting. As a result, the manufacturing of the air inlet housing 19 and the second air inlet pipe 192 is simple. The air inlet housing 19 and the second air inlet pipe 192 may also be separately arranged. For example, the air inlet housing 19 is removably connected to the second air inlet pipe 192 through threads, bolts, or a snap-fit. For another example, the air inlet housing 19 is fixedly connected to the second air inlet pipe 192 through bonding, welding, or the like. In this way, the manufacturing of each of the air inlet housing 19 and the second air inlet pipe 192 may be conveniently adjusted as desired.

It should be noted that formation of the second receiving cavity 112 by enclosing the bottom housing 17 and the mounting portion 40 means that cavity walls of the second receiving cavity 112 are partially or all formed by the bottom housing 17 and the mounting portion 40. In the embodiments, as long as the cavity walls of the second receiving cavity 112 are partially by the bottom housing 17, the opening 51 formed at the bottom housing 17 may be in communication with the second receiving cavity 112. In some embodiments, the housing 10 further includes the upper housing, and the second receiving cavity 112 may be formed by the upper housing, the bottom housing 17, and the mounting portion 40 together.

Referring to FIG. 13 and FIG. 14, an oil reduction cavity 27 is formed by an outer wall of the second air inlet pipe 192, an inner wall of the air inlet housing 19, and the bottom housing 17 together. The opening 51 and the third through hole 243 are each in communication with the oil reduction cavity 27. The airflow enters the oil reduction cavity 27 from the third through hole 243, and the oil reduction cavity 27 may adsorb oil fume particles that are possibly carried by the airflow. The airflow adsorbed by the oil reduction cavity 27 enters the second receiving cavity 112 through the opening 51. Therefore, a possibility that the oil fume particles enter the second receiving cavity 112 through the pressurizing channel 26 is reduced.

In some embodiments, referring to FIG. 12 and FIG. 13, the housing 10 is provided with a plurality of fins 18. Each of the plurality of fins 18 has a light-transmitting hole 181. The plurality of fins 18 is arranged at intervals in the second receiving cavity 112 along an optical axis of the light emitting assembly 31 and/or an optical axis of the light receiving assembly 32.

The plurality of fins 18 continuously changes an inner diameter of the second receiving cavity 112, allowing the oil fume particles entering the second receiving cavity 112 to be adsorbed on the plurality of fins 18. Moreover, a probability that the oil fume particles are in contact with the light emitting assembly 31 and/or the light receiving assembly 32 is reduced.

In some embodiments, the light-transmitting hole 181 of the fin 18 may be circular to shape the light emitted from the light emitting assembly 31 and/or the light received by the light receiving assembly 32. The plurality of fins 18 may be all connected to the mounting portion 40 or the bottom housing 17. The plurality of fins 18 may be partially connected to the mounting portion 40 or the bottom housing 17. In some embodiments, the housing 10 further includes the upper housing. The accommodation cavity 11 is formed by the upper housing and the bottom housing 17 together. The second receiving cavity 112 may be formed by the upper housing, the bottom housing 17, and the mounting portion 40 together. In this case, the plurality of fins 18 may be all connected to the upper housing or the bottom housing 17, or be partially connected to the upper housing or the bottom housing 17. In this way, the plurality of fins 18 may be engaged with each other as the upper housing is assembled with the bottom housing 17.

In some embodiments, referring to FIG. 12, the fin 18 may include an upper fin and a lower fin. An end of the upper fin is connected to the upper housing, and another end of the upper fin has a first groove. An end of the lower fin is connected to the bottom housing 17, and another end of the lower fin has a second groove. The first groove is spliced with the second groove to form the light-transmitting hole 181. The upper fin is assembled with the lower fin to form the light-transmitting hole 181. During manufacturing, the upper fin with the first groove and the lower fin with the second groove are manufactured respectively, to achieve convenient de-molding.

In some embodiments, referring to FIG. 11 and FIG. 12, the housing 10 further includes an upper housing and an exhaust pipe penetrating the upper housing. The accommodation cavity 11 is formed by the upper housing and the lower housing together. An exhaust cavity 16 is formed inside the exhaust pipe. The air outlet 22 is formed at a position communicating between the exhaust cavity 16 and the second receiving cavity 112. The upper housing may be separated from the lower housing to mount the sensor assembly 30 in the accommodation cavity 11. The upper housing may be assembled with the lower housing after the mounting is completed. In addition, when the sensor assembly 30 needs to be replaced, maintained, or the like, the upper housing may also be separated from the lower housing, which is convenient for the user to operate the sensor assembly 30.

In some embodiments, the upper housing may include an upper cover and an exhaust cover. The exhaust pipe may include a first exhaust pipe and a second exhaust pipe. The first exhaust pipe penetrates the upper cover. The second exhaust pipe penetrates the exhaust cover. An interior of the first exhaust pipe is in communication with an interior of the second exhaust pipe to form an exhaust cavity 16. The accommodation cavity 11 is formed by the upper cover, the first exhaust pipe, and the lower housing together. Further, refer to FIG. 11, an axis of the air inlet cavity 101 and an axis of the exhaust cavity 16 are located on one straight line. After the oil fumes enter the accommodation cavity 11 from the air inlet cavity 101, the oil fumes may enter the exhaust cavity 16 without changing its movement direction. As a result, the probability that the oil fume escapes in the accommodation cavity 11 can be reduced.

Referring to FIG. 23, an embodiment of the present disclosure provides a kitchen appliance 1000. The kitchen appliance 1000 includes a casing 200, a fan assembly 300, and an oil fume sensor 100. The fan assembly 300 is mounted in the casing 200.

According to the kitchen appliance 1000 in the embodiments of the present disclosure, the air inlet cavity 101 is tapered to allow a speed at which the oil fumes entering the accommodation cavity 11 from the air inlet cavity 101 is constantly accelerated as a volume of the air inlet cavity 101 gradually decreases. In this way, the oil fumes are accelerated to pass through the accommodation cavity 11 and quickly discharged from the air outlet 22. As a result, it is possible to reduce dissipation of the oil fumes in the accommodation cavity 11. Therefore, the probability that the oil fumes are condensed in the sensor assembly 30 is lowered, and the service life and performance of the oil fume sensor are improved.

In an example of FIG. 23, the kitchen appliance 1000 is an upper-discharge kitchen appliance 1000. It can be understood that, in other embodiments, the kitchen appliance 1000 may be a lower-discharge kitchen appliance 1000 or a side-discharge kitchen appliance 1000, and the present disclosure is not limited in this regard. As an example, detailed descriptions will be set forth by taking the upper-discharge kitchen appliance 1000 as the kitchen appliance 1000. In some embodiments, the kitchen appliance 1000 includes, but is not limited to, a range hood, an integrated cooker, or other electric appliances having an oil fume discharge function. As an example, the description will be provided taking the range hood the kitchen appliance 1000. The range hood may be a variable frequency range hood.

The kitchen appliance 1000 according to the embodiments of the present disclosure includes, but is not limited to, a deflector assembly and a check valve. The casing 200 is disposed on the deflector assembly. The deflector assembly has a smoke collecting cavity and a plurality of function buttons. The oil mesh and the top plate are provided in the smoke collecting cavity, and the plurality of function buttons may be used for the user to input the operation instruction. The fan assembly 300 is provided in the casing 200. The fan assembly 300 includes a volute and a fan, and has an air inlet and an air outlet. The fan is arranged in the volute. A volute air duct is formed in the volute. The air inlet is configured to allow the oil fumes to enter the fan assembly 300. The air outlet is in communication with the volute air duct and is configured to allow the oil fumes to be discharged out of the fan assembly 300. The check valve is connected to the top of the casing 200. The check valve internally has a check valve air duct. It can be understood that the check valve refers to a valve that an openable/closable member is a circular valve clack and is configured to block backflow of a medium by means of its own weight and a pressure of the medium. The check valve may be a lifting check valve and a swing check valve.

The oil fume sensor 100 is arranged at the position on the kitchen appliance 1000 through which the gas containing the oil fumes passes, such as a center of the deflector and an air inlet of the fan assembly 200. In this way, the gas containing the oil fumes may be allowed for entering the oil fume sensor 100 for detection. After the oil fumes are generated around the kitchen appliance 1000, the oil fumes move under the action of the household appliance 1000 and enter the air inlet cavity 101 of the oil fume sensor 100 arranged in the casing 200. The light emitting assembly 31 can emit light to the accommodation cavity 11. The gas in the accommodation cavity 11 affects the light emitted from the light emitting assembly 31. The light receiving assembly 32 can receive the light affected by the gas in the accommodation cavity 11 and outputs the electrical signal based on the received light. The detected gas is discharged from the oil fume sensor 100 through the air outlet 22 and the exhaust cavity 16.

In some embodiments, referring to FIG. 23, the oil fume sensor 100 is arranged at the air inlet of the fan assembly 300. In this way, thus airflow is concentrated, and the oil fume particles have a high concentration without being removed by a centrifugal effect of the fan assembly 300. Therefore, a distinguishing capability of the oil fume sensor 100 is lowered. In addition, a distance from the oil fume particles is short, which can quickly sample the oil fumes.

The air inlet of the fan assembly 300 is formed at the volute. The airflow enters the volute air duct from the air inlet formed at the volute. The airflow in the volute air duct is discharged from the air outlet by the fan. In some embodiments, compared with other positions, the air inlet of the fan assembly 300 has a small pressure, which is convenient to suck the air into the fan assembly 300. Therefore, the oil fume sensor 100 is arranged at the air inlet of the fan assembly 300. Moreover, it is also possible for a pressure difference to be generated between the air inlet 21 and the air outlet 22 of the oil fume sensor 100. In combination with a tapered shape of the air inlet cavity 211, the speed at which the oil fume passes through the accommodation cavity 11 is further accelerated, and the probability that the oil fume escapes in the accommodation cavity 11 is reduced.

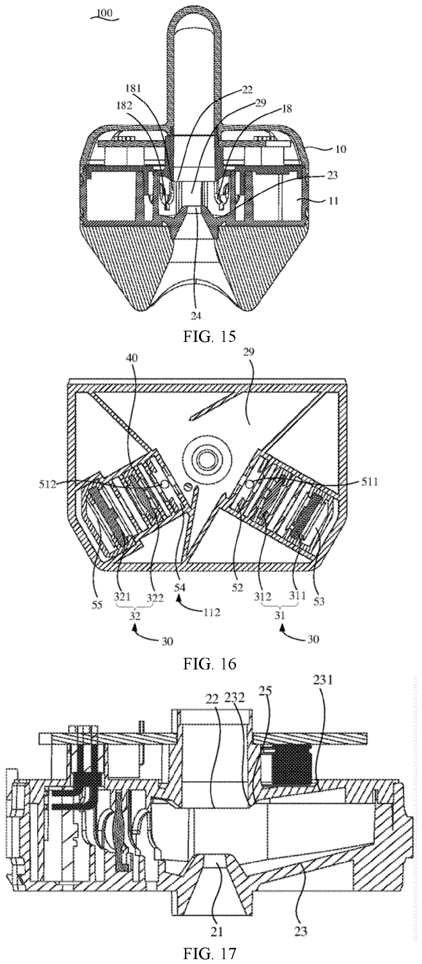

Referring to FIG. 15 and FIG. 16, an embodiment of the present disclosure provides an oil fume sensor 100. The oil fume sensor 100 includes a housing 10 and a sensor assembly 30. The housing 10 has an accommodation cavity 11. A detection cavity 29 is formed a position where the accommodation cavity 11 contacts oil fumes. The detection cavity 29 has a inclined guide surface 23. A through hole 24 is formed at a lower cavity wall of the detection cavity 29. The inclined guide surface 23 is configured to guide an oil drop to be discharged out of the detection cavity 29 through the through hole 24. The sensor assembly 30 is located in the accommodation cavity 11.

According to the oil fume sensor 100 in the embodiments of the present disclosure, the inclined guide surface 23 is formed in the detection cavity 29. In this way, the oil drop moves on the inclined guide surface 23 to be guided to the through hole 24 and discharged out of the detection cavity 29. As a result, it is possible to prevent the oil drop from be collected in the detection cavity 29, which would block the sensor assembly 30 from detecting the oil fumes. Therefore, detection accuracy of the sensor assembly 30 can be ensured.

The housing 10 is in various shapes, such as a cuboid, a sphere, a vertebral body shape, or other shapes, and the present disclosure is not limited in this regard. In an embodiment, the housing 10 is of a shuttle shape. That is, an outer diameter of the housing 10 gradually increases from top to bottom and then gradually decreases from top to bottom. In this way, an oil drop condensed at the outer wall of the housing 10 may naturally drip under the gravity. Moreover, the speed at which the outer wall of the housing 10 is contaminated by the oil stains is reduced.

The accommodation cavity 11 may be in various shapes, such as a cuboid, spheroidal, pyramidal shape, or other shapes. The shape of the accommodation cavity 11 may follow the shape of the housing 10, and the present disclosure is not limited in this regard. There may be a position at the accommodation cavity 11 where the accommodation cavity 11 is in contact with the oil fumes. That is, the accommodation cavity 11 may include a detection cavity 29. In addition, there may be a position at the accommodation cavity 11 where the accommodation cavity 11 is spaced apart from the oil fumes, and the sensor assembly 30 may be mounted at this position to protect the sensor assembly 30 from coming into contact with the oil fumes, or a shape of the detection cavity 29 may be altered at this position, and the present disclosure is not limited in this regard.

The detection cavity 29 is in contact with the oil fumes. In this way, the oil fumes are easily condensed into the oil drop in the detection cavity 29. Therefore, the inclined guide surface 23 is formed in the detection cavity 29 to guide the oil drop to leave from the detection cavity 29. It should be noted that the detection cavity 29 refers to a part where it is considered in the design that the detection cavity 29 will be in contact with the oil fumes. For example, when a part where the sensor assembly 30 is partially accommodated in the accommodation cavity 11 is deemed to be not in contact with the oil fumes as designed, this part is not regarded as the detection cavity 29. When sealing of the space where the sensor assembly 30 is partially accommodated in the accommodation cavity 11 is deemed to fail potentially, this part is finally in contact with the oil fumes, and is considered as the detection cavity 29. Moreover, the inclined guide surface 23 and the through hole 24 may also be formed at this part to guide the oil fume to flow out.

The inclined guide surface 23 is configured to guide the oil drop. The inclined guide surface 23 has many arrangement positions. The inclined guide surface 23 may be arranged at an upper cavity wall of the detection cavity 29, at the lower cavity wall of the detection cavity 29, or at a side cavity wall of the detection cavity 29, as long as the inclined guide surface 23 can guide the oil drop towards the through hole 24, and the present disclosure is not limited in this regard.

The inclined guide surface 23 has various inclination angles, as long as a predetermined angle is formed between inclined guide surface 23 and a horizontal plane. The inclination angles of various values may be formed between the inclined guide surface 23 and the horizontal plane may exist. For example, the inclination angle may be 5°, 10°, 15°, 20°, 25°, 50°, 70°, 90°, or the like, and the embodiments do not enumerate this herein. It's worth noting that the inclined guide surface 23 may extend in the vertical direction. That is, the through hole 24 may be located directly below the inclined guide surface 23. Therefore, the inclined guide surface 23 extends in the vertical direction, and thus can guide the oil drop to drip into the through hole 24 and then discharged out of the detection cavity 29 through the through hole 24.

The inclined guide surface 23 may have various structure. The inclined guide surfaces 23 may be planar, or have several oil guide grooves extending towards the through hole 24, and the present disclosure is not limited in this regard. A plurality of inclined guide surfaces 23 may be provided. One, two, three, ten, or twenty inclined guide surfaces 23 may be formed in the detection cavity 29, and the number of inclined guide surfaces 23 may be adjusted based on factors such as a size of the inclined guide surface 23 and manufacturing convenience, and the present disclosure is not limited in this regard.

A plurality of through holes 24 may be provided. The lower cavity wall of the detection cavity 29 may have one, two, three, four, five, ten, or more through holes 24, and the embodiments do not enumerate this herein. It should be noted that the through hole 24 is arranged at the lower cavity wall of the detection cavity 29, allowing the oil drop to drip from the through hole 24 under the gravity.

The through hole 24 may have one-to-one correspondence to the inclined guide surface 23. A plurality of through holes 24 may correspond to one inclined guide surface 23. For example, the plurality of through holes 24 are formed at one inclined guide surface 23 and is different in height. In this way, a small amount of oil drops may be discharged from the through holes 24 at higher positions, and more oil drops are gathered to the through holes 24 at lower positions and discharged from these through holes 24. In addition, one through hole 24 may correspond to a plurality of inclined guide surfaces 23. For example, the inclined guide surface 23 is formed at the lower cavity wall of the detection cavity 29 to guide the oil drop at the lower cavity wall of the detection cavity 29 to the through hole 24 to be discharged from the through hole 24. Further, the inclined guide surface 23 corresponding to the through hole 24 is also formed at the upper cavity wall of the detection cavity 29, to guide the oil drop at the upper cavity wall of the detection cavity 29 to the through hole 24 to be discharged from the through hole 24.

The through holes 24 may be in various shapes. A cross sections of the through hole 24 may be circular, triangular, elliptical, or the like, and the embodiments do not enumerate this herein.

The oil fume sensor 100 may be an infrared detection sensor, a laser detection sensor, or the like, and the present disclosure is not limited in this regard. A detailed description will be provided by taking the infrared detection sensor as the oil fume sensor 100.

The sensor assembly 30 may include a light emitting assembly 31 and a light receiving assembly 32. The light emitting assembly 31 is configured to emit light to the detection cavity 29. The light receiving assembly 32 is configured to receive the light emitted from the light emitting assembly 31 and output the electrical signal based on the received light. Generally, the particle size of the oil fume particles is in the range of 100nm to 10µm. In an embodiment, when the oil fume particles pass through the light path of the infrared light emitted from the light emitting assembly 31, the infrared light may be shielded, scattered, and diffracted. That is, oil fumes particles of the detection cavity 29 will affect the intensity at which the light receiving assembly 32 receives the light emitted from the light emitting assembly 31 to change the light received by the light receiving assembly 32, thereby determining the concentration of the oil fumes particles based on this change.

In some embodiments, referring to FIG. 15, FIG. 16, and FIG. 18, the through hole 24 includes a first through hole 241. The housing 10 has an air inlet 21 and an air outlet 22. The air inlet 21 and the air outlet 22 are each in communication with the detection cavity 29. The inclined guide surface 23 is formed at the lower cavity wall of the detection cavity 29. The air inlet 21 serves as the first through hole 241. With this arrangement, when the air flows through the air inlet 21, the air inlet 21 can function as the through hole 24. That is, the oil drop can be discharged out of the detection cavity 29 through the air inlet 21. Therefore, the structure is simple.

In some embodiments, airflow of the air inlet 21 enters the detection cavity 29. The air outlet 22 is used for allowing for an outflow of the airflow in the detection cavity 29 from the detection cavity 29. The air outlet 22 may be formed at several positions. The air outlet 22 may be located at the upper cavity wall of the detection cavity 29, at the lower cavity wall of the detection cavity 29, or at a left cavity wall or a right cavity wall of the detection cavity 29, and the present disclosure is not limited in this regard.

The air inlet 21 and the air outlet 22 may be located at an upper end and a lower end of the detection cavity 29. The air inlet 21 is located directly below the air outlet 22. Since the oil fume particles generally move upwards, the oil fume particles entering the detection cavity 29 from the air inlet 21 can thus naturally move to the air outlet 22 to be discharged from the air outlet 22. The probability that the oil fume particles escape in the detection cavity 29 is further lowered. In addition, the movement positions of the oil fume particles are concentrated, which is more conducive for the detection of the oil fume particles by the sensor assembly 30.

It should be noted that a height of the air inlet 21 may be lower than a height of a part of the inclined guide surface 23 at its lowest position. For example, the air inlet 21 may be located at the lowest position of the inclined guide surface 23. Moreover, the inclined guide surface 23 can guide the oil drop to the lowest position of the inclined guide surface 23. Therefore, the oil drop slides to the air inlet 21 under the action of gravity. The height of the air inlet 21 may be higher than the height of the part of the inclined guide surface 23 at its lowest position. For example, the air inlet 21 may be located at the lowest position of the inclined guide surface 23. Moreover, the inclined guide surface 23 guides the oil drop to the lowest position of the inclined guide surface 23. Therefore, the oil drops are gathered at the lowest position of the inclined guide surface 23, and finally spill over the air inlet 21, and then is discharged from the detection cavity 29 through the air inlet 21.

Further, the through hole 24 further includes a second through hole 242. The a second through hole 242 is formed at the lower cavity wall of the detection cavity 29 and spaced apart from the first through hole 241. The oil drop is discharged from the detection cavity 29 through the first through hole 241 and the second through hole 242 together. Therefore, it is possible to short a path along which the oil drop pass. Therefore, the oil drop may be discharged along a path as short as possible. Moreover, a probability that the oil drop is adhered to the inclined guide surface 23 is lowered.

In a case where the lower cavity wall of the detection cavity 29 only has one air inlet 21 to allow for an outflow of the oil drop from the detection cavity 29, an oil drop away from the air inlet 21 needs to flow into the air inlet 21 along a long path, which increases the probability that the oil drop is adhered to the inclined guide surface 23. Therefore, the first through hole 241 and the second through hole 242 are formed at the lower cavity wall of the detection cavity 29, thereby shorting a distance between the oil drop and the through hole 24.

In some embodiments, an inclination angle of the inclined guide surface 23 relative to a horizontal plane is greater than or equal to 10°.

Therefore, the inclined guide surface 23 can effectively guide the movement of the oil drop.

In some embodiments, viscosity of the oil drop is large. Therefore, the movement of the oil drop can be better guided only by a large inclination angle.

In some embodiments, referring to FIG. 15 and FIG. 17, the inclined guide surface 23 includes a first inclined guide surface 231 formed at an upper cavity wall of the detection cavity 29. Therefore, it is possible to guide the oil drop at the upper cavity wall of the detection cavity 29. Moreover, the oil drop at the upper cavity wall of the detection cavity 29 can be discharged from the detection cavity 29 through the through hole 24.

In some embodiments, the lowest position of the first inclined guide surface 231 may directly face towards the through hole 24. That is, the oil drop drips from the upper cavity wall of the detection cavity 29 at the lowest position of the first inclined guide surface 231, and can just fall into the through hole 24, and then is discharged from the detection cavity 29 through the through hole 24. The inclined guide surface 23 may also be formed at the lower cavity wall of the detection cavity 29. In this case, the lowest position of the first inclined guide surface 231 may also correspond to the inclined guide surface 23 formed at the lower cavity wall of the detection cavity 29. That is, the oil drop at the lowest position of the first inclined guide surface 231 drops from the upper cavity wall of the detection cavity 29 to the inclined guide surface 23 at the lower cavity wall of the detection cavity 29, and is guided by the inclined guide surface 23 at the lower cavity wall of the detection cavity 29 to be discharged from the detection cavity 29 through the through hole 24. The present disclosure is not limited in this regard.

Further, referring to FIG. 15 and FIG. 17, the housing 10 has an air inlet 21 and an air outlet 22. The air inlet 21 and the air outlet 22 are each in communication with the detection cavity 29. The inclined guide surface 23 includes a second inclined guide surface 232. The air outlet 22 is located on the upper cavity wall of the detection cavity 29. A guide protrusion 25 protrudes from a periphery of the air outlet 25. The second inclined guide surface 232 is formed at a periphery of the guide protrusion 25, and an inclination angle of the second inclined guide surface 32 is greater than an inclination angle of the first inclined guide surface 231.

The oil drop is more easily dropped on the second inclined guide surface 232 with a large inclination angle. Therefore, it is possible to relieve a problem that the oil drop is adhered to the first inclined guide surface 231 or to the second inclined guide surface 232.

In some embodiments, the guide protrusion 25 may be formed in various manners. The guide protrusion 25 and the housing 10 may be integrally formed. For example, the guide protrusions 25 and the housing 10 are formed through injection molding. In addition, for example, the guide protrusions 25 and the housing 10 are formed through simultaneous cutting. Therefore, the guide protrusion 25 and the housing 10 are simple to be manufactured. In addition, a gap generated by no connection between the guide protrusion 25 and the housing 10. Therefore, it is possible to prevent the oil drop from entering between the guide protrusion 25 and the housing 10, which is difficult to be cleaned.

The guide protrusion 25 may also be separated from the housing 10. For example, the guide protrusion 25 may be removably connected to the housing 10 by means of bolts, through snap-fit, or the like. In this way, replacement of the guide protrusion 25 is convenient. Moreover, manufacturing steps or manufacturing manners of the guide protrusion 25 and the housing 10 or the like may be conveniently adjusted based on manufacturing requirements. Further, for example, the guide protrusion 25 may also be fixedly connected to the housing 10 by means of bonding, through welding, or the like. In this way, the manufacturing steps and manufacturing manners of the guide protrusion 25 and the housing 10 can be conveniently adjusted based on manufacturing requirements.

It should be noted that the inclined guide surface 23 may also be formed at an inner edge of the guide protrusion 25 to guide the oil drop flowing through the air outlet 22.

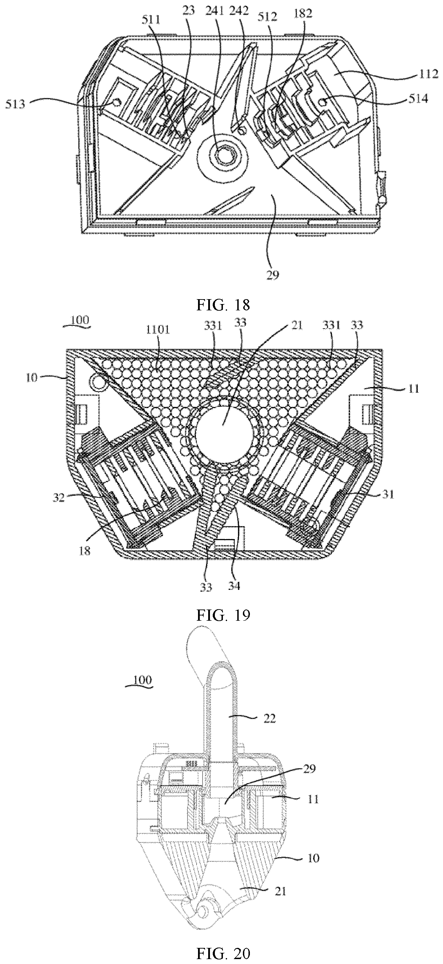

In some embodiments, referring to FIG. 16 and FIG. 18, the sensor assembly 30 includes a light emitting assembly 31 and a light receiving assembly 32. A mounting portion 40 is disposed in the accommodation cavity 11. A second receiving cavity 112 is formed by the mounting portion 40 and the housing 10. The light emitting assembly 31 and/or the light receiving assembly 32 are mounted in the second receiving cavity 112. An opening is formed at a lower cavity wall of the second receiving cavity 112, and the opening serves as the through hole 24.

The mounting portion 40 can protect the light emitting assembly 31 and/or the light receiving assembly 32, and thus it is possible to lower a probability that the light emitting assembly 31 and/or the light receiving assembly 32 are in contact with the oil fumes. In addition, the opening is formed at the second receiving cavity 112 to serve as the through hole 24, which facilitates dropping of oil drop condensed in the second receiving cavity 112.

In some embodiments, the mounting portion 40 may be formed in various manners. The mounting portion 40 may be formed by assembling the plurality of mounting plates, or formed by turning on the material, and the present disclosure is not limited in this regard.

The light emitting assembly 31 may be mounted in the second receiving cavity 112 or in the second receiving cavity 112, or both the light emitting assembly 31 and the light receiving assembly 32 are mounted in the second receiving cavity 112, and the present disclosure is not limited in the regard.

The second receiving cavity 112 may have several structures. The second receiving cavity 112 may have an integrated structure, or may be formed by several separate parts, and the present disclosure is not limited in the regard.

Further, referring to FIG. 15 to FIG. 18, the housing 10 includes a plurality of fins 18. The plurality of fins 18 is arranged at intervals in the second receiving cavity 112 along an optical axis of the light emitting assembly 31 and/or an optical axis of the light receiving assembly 32. Each of the plurality of fins 18 has a light-transmitting hole 181 and a liquid flowing groove 182 in communication with the light-transmitting hole 181. The inclined guide surface 23 is formed at a wall of the liquid flowing groove 182.

The plurality of fins 18 continuously changes the inner diameter of the second receiving cavity 112, to allow the oil fume particles entering the second receiving cavity 112 to be adsorbed on the plurality of fins 18. Moreover, the probability that the oil fume particles are in contact with the light emitting assembly 31 and/or the light receiving assembly 32 is lowered. In addition, the inclined guide surface 23 is formed at the liquid flowing groove, which is beneficial for an oil drop on the plurality of fins 18 to enter the through hole 24 and then be discharged from the detection cavity 29.

In some embodiments, a light-transmitting through hole 24 of the fin 18 may be circular, to shape the light emitted from the light emitting assembly 31 and/or the light received by the light receiving assembly 32.

The housing 10 may include an upper housing, an exhaust pipe penetrating the upper housing, a lower housing, and an air inlet pipe penetrating the lower housing. The accommodation cavity 11 is formed by the upper housing and the lower housing. The air inlet 21 is located in the air inlet pipe. The air outlet 22 is located in the exhaust pipe. The mounting portion 40 may be fixedly connected to the lower housing. The second receiving cavity 112 is formed by the mounting portion 40, the lower housing, and the upper housing together. When the upper housing and the lower housing are assembled together, the second receiving cavity 112 is closed to protect the sensor assembly 30 mounted in the second receiving cavity 112. When the upper housing is separated from the lower housing, the second receiving cavity 112 is in communication with the external environment, which is convenient to mount, disassemble, and maintain the sensor assembly 30.

The lower housing and the air inlet pipe may be integrally formed through injection molding. For example, the lower housing and the air inlet pipe are formed through simultaneous cutting. As a result, the manufacturing of each of the lower housing and the air inlet pipe is simple. The lower housing may be of various shapes, such as a cuboid shape, a semi-sphere shape, or the like, and the present disclosure is not limited in this regard. The air inlet pipe may extend in the vertical direction, in the horizontal direction, or in a direction at any angle relative to the vertical direction. In an embodiment, the air inlet pipe extends in the vertical direction. Therefore, the oil drop condensed in the air inlet pipe can drip under the gravity, to reduce the speed at which the air inlet pipe is contaminated by the oil stains and prolong the service life of the air inlet pipe. The air inlet pipe has various shapes, and its cross section may be in a square, circular shape, or the like, and the present disclosure is not limited in this regard. The upper housing and the exhaust pipe are similar to the lower housing and the air inlet pipe, and the detailed description thereof will be omitted herein.

The plurality of fins 18 may be all connected to the mounting portion 40, the lower housing, or the upper housing, or be partially connected to the upper housing or the lower housing 17. In this way, the plurality of fins 18 may be engaged with each other when the upper housing is assembled with the lower housing.

The fin 18 may include an upper fin and a lower fin. An end of the upper fin is connected to the upper housing, and another end of the upper fin has a first groove. An end of the lower fin is connected to the lower housing, and another end of the lower fin has a second groove. The first groove is spliced with the second groove to form the light-transmitting through hole 24.

The upper fin is assembled with the lower fin to form the light-transmitting through hole 24. During manufacturing, the upper fin with the first groove and the lower fin with the second groove are manufactured respectively, thereby providing convenient de-molding.

In some embodiments, referring to FIG. 16 and FIG. 18, the light emitting assembly 31 includes a light emitter 311 and a transmitting lens 312 mounted in several spaces between the plurality of fins 18. The second receiving cavity 112 includes a first cavity 52 and a second cavity 53. The first cavity 52 is separated from the second cavity 53 by the transmitting lens 312. The light emitter 311 is mounted in the second cavity 53. The opening includes a first opening 511 formed at a lower cavity wall of the first cavity 52.

In this way, the first cavity 52 is separated from the second cavity 53 by the transmitting lens 312 to protect the light emitter 311 mounted in the second cavity 53. Thus, it is possible to prevent the light emitter 311 from being in contact with the oil fumes. In addition, the light emitter 311 can emit parallel light into the detection cavity 29 through the transmitting lens 312.

In some embodiments, since the transmitting lens 312 can protect the second cavity 53 to allow the second cavity 53 not to be substantially in contact with the oil fumes, the opening is formed at the lower cavity wall of the first cavity 52, thereby allowing an oil drop condensed in the first cavity 52 to be discharged through the first opening 511.

It can be understood that the transmitting lens 312 is a convex lens, and the light emitter 311 is mounted at a focal point of the transmitting lens 312.

In some embodiments, referring to FIG. 16 and FIG. 18, the light receiving assembly 32 includes a light receiver 321 and a receiving lens 322. The receiving lens 322 is mounted in several spaces between the plurality of fins 18. The second receiving cavity 112 includes a third cavity 54 and a fourth cavity 55. The third cavity 54 is separated from the fourth cavity 55 by the receiving lens 322. The light receiver 321 is mounted in the fourth cavity 55. The opening includes a second opening 512 formed at a lower cavity wall of the third cavity 54.

In this way, the third cavity 54 is separated from the fourth cavity 55 by the receiving lens 322 to protect the light receiver 321 mounted in the fourth cavity 55. Thus, it is possible to prevent the light receiver 321 from being in contact with the oil fumes. In addition, the light receiver 321 can receive the light in the detection cavity 29 through the receiving lens 322.

In some embodiments, since the receiving lens 322 can protect the fourth cavity 55 to allow the fourth cavity 55 not to be substantially in contact with the oil fumes, the opening is formed at the lower cavity wall of the third cavity 54, thereby allowing an oil drop condensed in the third cavity 54 to be discharged from the second opening 512.

It can be understood that the receiving lens 322 is a convex lens, and the light receiver 321 is mounted at a focal point of the receiving lens 322.

Further, referring to FIG. 18, the opening includes a third opening 513 formed at a lower cavity wall of the second cavity wall 53.

In a case where sealing of the second cavity 53 fails and the second cavity 53 is in contact with the oil fumes, an oil drop in the second cavity 53 can be discharged from the third opening 513.

In some embodiments, referring to FIG. 18, the opening includes a fourth opening 514 formed at a lower cavity wall of the fourth cavity wall 55. In a case where sealing of the fourth cavity 55 fails and the fourth cavity 55 is in contact with the oil fumes, an oil drop in the fourth cavity 55 can be discharged from the fourth opening 514.

An embodiment of the present disclosure provides a kitchen appliance 1000. The kitchen appliance 1000 includes a casing 200, a fan assembly 300, and an oil fume sensor 100. The fan assembly 300 is mounted in the casing 200.

In the kitchen appliance 1000 according to the embodiments of the present disclosure, the inclined guide surface 23 is formed in the detection cavity 29. In this way, the oil drop moves on the inclined guide surface 23 to be guided to the through hole 24 and discharged from the detection cavity 29. Therefore, it is possible to prevent the oil drop from gathering in the detection cavity 29, and block the sensor assembly 30 from detecting the oil fumes. Therefore, the detection accuracy of the sensor assembly 30 can be ensured.

In some embodiments, in the example shown in FIG. 23, the kitchen appliance 1000 is an upper-discharge kitchen appliance 1000. It can be understood that, in other embodiments, the kitchen appliance 1000 may be a lower-discharge kitchen appliance 1000 or a side-discharge kitchen appliance 1000, and the present disclosure is not limited herein. As an example, detailed descriptions will be set forth by taking the upper-discharge kitchen appliance 1000 as the kitchen appliance 1000. In some embodiments, the kitchen appliance 1000 includes, but is not limited to, a range hood, an integrated cooker, or other electric appliances having an oil fume discharge function. As an example, the description will be provided taking the range hood the kitchen appliance 1000. The range hood may be a variable frequency range hood.

The kitchen appliance 1000 according to the embodiments of the present disclosure includes, but is not limited to, a deflector assembly and a check valve. A casing 200 is disposed on the deflector assembly. The deflector assembly has a smoke collecting cavity and a plurality of function buttons. An oil mesh and a top plate are provided in a smoke collecting cavity, and the plurality of function buttons may be used for the user to input the operation instruction. The fan assembly 300 is provided in the casing 200. The fan assembly 300 includes a volute and a fan, and has an air inlet and an air outlet. The fan is arranged in the volute. A volute air duct is formed in the volute. The air outlet is in communication with the volute air duct and is configured to allow the oil fumes to be discharged out of the fan assembly 300. The check valve is connected to the top of the casing 200. The check valve internally has a check valve air duct. It can be understood that the check valve refers to a valve that an openable/closable member is a circular valve clack and is configured to block backflow of a medium by means of its own weight and a pressure of the medium. The check valve may be a lifting check valve and a swing check valve.

The oil fume sensor 100 may be arranged at a position on the kitchen appliance 1000 through which the gas containing the oil fumes passes, such as a center of the deflector and an air inlet of the fan assembly 200. In this way, the gas containing the oil fumes may be allowed for entering the oil fume sensor 100 for detection.

In some embodiments, the oil fume sensor 100 is arranged at the air inlet of the fan assembly 300.

In this way, the airflow at the air inlet of the fan assembly 300 is concentrated, and the oil fume particles have the high concentration without being removed by the centrifugal effect of the fan assembly 300. Therefore, the distinguishing capability of the oil fume sensor 100 is lowered. In addition, the distance from the oil fume particles is short, which can quickly sample the oil fumes.

The air inlet of the fan assembly 300 is formed at the volute. The airflow enters the volute air duct from the air inlet formed at the volute. The airflow in the volute air duct is discharged by the fan through the air outlet.

Referring to FIG. 19 and FIG. 20, an embodiment of the present disclosure provides an oil fume sensor 100. The oil fume sensor 100 includes a housing 10, a light emitting assembly 31, a light receiving assembly 32, and a plurality of traps 33. The housing 10 has an air inlet 21, an air outlet 22, and an accommodation cavity 11. The air inlet 21 and the air outlet 22 are each in communication with the accommodation cavity 11. The light emitting assembly 31 is configured to emit light to the accommodation cavity 11. The light receiving assembly 32 is configured to receive the light emitted from the light emitting assembly 31. The plurality of light traps 33 are provided in the accommodation cavity 11. Each of the plurality of light traps 33 has a light trap cavity 331 for removing light emitted into the light trap cavity 331.

In the oil fume sensor 100 according to the embodiments of the present disclosure, by providing the light trap 33 to form the light trap cavity 331, the light emitted to the light trap cavity 331 can be reflected and absorbed in the light trap cavity 331 for many times, which can lower a probability that the light receiving assembly 32 receives the light reflected by the cavity wall of the accommodation cavity 11. Therefore, it is possible to effectively eliminate an error of detection of the light receiving assembly 32 for the concentration of the oil fumes.

In some embodiments, the oil fume sensor 100 may be a device for detecting the gas particle concentration. Moreover, the oil fume particles generated by the user using the kitchen appliance 1000 may be mixed in the gas and enter the oil fume sensor 100 together. Therefore, it may be determined that whether the user is cooking by detecting the gas particle concentration. A wide variety of device for detecting the gas particle concentration is available, which may be an infrared detection device, a laser detection device, or the like, and the present disclosure is not limited in this regard.

Taking the infrared detection device as the oil fume sensor 100, the oil fume sensor 100 may further include a main control board. The light emitting assembly 31 is configured to emit light to the gas possibly adhered with the oil fumes. The light receiving assembly 32 is configured to receive the light emitted from the light emitting assembly 31. The main control board 35 is electrically connected to the light emitting assembly 31 and the light receiving assembly 32. Therefore, the light emitting assembly 31 can be controlled to emit light. Moreover, a current condition in which the oil fumes are adhered to the gas can be determined based on light information received by the light receiving assembly 32. It can be understood that the particle size of the oil fume particles is in the range of 100 nm to 10 µm. When the oil fume particles pass through a light path of the light emitting assembly 31, shielding, scattering, and diffraction of the infrared light may be caused. That is, the oil fume particles can affect an intensity at which the light is received by the light receiving assembly 32 to change the light information obtained by the main control board.

An optical axis of the light emitting assembly 31 of the infrared detection device and an optical axis of the light receiving assembly 32 of the infrared detection device are located on one straight line. Therefore, it is possible to allow the light emitting assembly 31 to be arranged opposite to the light receiving assembly 32. At this time, in a case where there is no oil fumes particles 70, the light emitted from the light emitting assembly 31 may be received by the light receiving assembly 32 without shielding. In a case where there are the oil fumes particles 70, the oil fumes particles 70 may cause the shielding, scattering, and diffraction of the infrared light, and thus the light emitted from the light emitting assembly 31 cannot be completely received by the light receiving assembly 32. In this case, by providing the light traps 33, more accurate light information of the light receiving assembly 32 can be obtained in the case where there is no oil fumes particles 70, and the light that is not scattered to the light receiving assembly 32 can be absorbed by the oil fumes particles 70 in the case where there are the oil fumes particles 70. Therefore, the light received by the light receiving assembly 32 is mainly affected by the oil fumes particles 70.

An angle may be formed between the optical axis of the light emitting assembly 31 of the infrared detection device and the optical axis of the light receiving assembly 32 of the infrared detection device. In this case, the light emitted from the light emitting assembly 31 may not be received by the light receiving assembly 32 in the case where there is no oil fume particles 70. In the case where there are the oil fume particles 70, the oil fume particles 70 can cause the shielding, scattering, and diffraction of the infrared light, and thus the light emitted from the light emitting assembly 31 can only be partially received by the light receiving assembly 32. In this case, by providing the light traps 33, in the case where there is no oil fume particles 70, the light emitted from the light emitting assembly 31 can be absorbed to prevent error of the light information from being generated due to the reflected light received by the light receiving assembly 32, and in the case where there are the oil fume particles 70, the light that is not scattered to the light receiving assembly 32 can be absorbed. Therefore, the light received by the light receiving assembly 32 to be mainly affected by the oil fumes particles 70.

The main control board 35 may also be provided with a communication module. The communication module may be connected to a mobile terminal such as a mobile phone, a tablet, and a computer, which is convenient for the user to control operation of the oil fume sensor 100. Moreover, the communication module may also be electrically connected to or in a communication connection with other components of the kitchen appliance 1000, to facilitate opening or closing the oil fume sensor 100 based on the state of the switch of the kitchen appliance 1000, and opening and closing the motor or increasing and reducing the power of the motor based on the detection result of the oil fume sensor 100.

The housing 10 may include a bottom housing and an upper cover. The air inlet 21 is formed at the bottom housing. The air outlet 22 is formed at the upper cover. The accommodation cavity 11 is formed by the bottom housing and the upper cover. The bottom housing and the upper cover may be connected in various manners. For example, the bottom housing may be connected to the upper cover through threads. In an embodiment, a threaded protrusion is provided at a periphery of the bottom housing. A threaded groove is formed at an inner edge of the upper cover and is engaged with the threaded protrusion. The bottom housing and the upper cover each have a circular cross section. The upper cover is rotated relative to the bottom housing to realize mounting and dismounting of the upper cover rotates and the bottom housing. In this way, the upper cover is removably connected to the bottom housing, which facilitates installation, disassembly, and maintenance of each component in the accommodation cavity 11. In addition, the external oil fume particles 70 are difficult to enter the accommodation cavity 11 through a connection between the upper cover and the bottom housing. Further, for example, the bottom housing may be connected to the upper cover through an engagement between the snap and the through hole. In an embodiment, the snap protrudes from the upper cover, and the bottom housing has a through hole engaged with the snap. The snap is in a snap-fit with the through hole, to achieve the connection between the bottom housing and the upper cover, and the embodiments do not enumerate this herein.

The housing 10 may further include a plurality of fins 18. The plurality of fins 18 are respectively mounted at the bottom housing and the upper cover, and are located in the accommodation cavity 11. A semicircular through hole is formed at an end of the fin 18. A fin 18 mounted at the bottom housing and the fin 18 mounted at the upper cover may be assembled together when the bottom housing and the upper cover are connected to each other, thereby forming a circular through hole. In this way, the light emitted from the light emitting assembly 31 or the light received by the light receiving assembly 32 can be shaped by the circular through hole. In addition, each of the plurality of fins 18 only has the semicircular through hole, and two fins 18 are assembled to form the circular through hole. Therefore, manufacturing and de-molding of the fins 18 can be facilitated. Moreover, manufacturing efficiency is improved.

A wide variety of shapes for the light trap 33 is available. The light trap 33 may be of a cuboid shape, a cone shape, or the like, and the present disclosure is no limited herein. The shapes of the plurality of light traps 33 may be the same or different, and the detailed description thereof will be omitted herein.

It should be noted that there are various arrangement in which the light trap 33 has the light trap cavity 331. For example, the light trap 33 may have a recess forming the light trap cavity 331, or the light trap cavity 331 may be formed by assembling the light trap 33 and the cavity wall of the accommodation cavity 11 together, or the light trap cavity 331 may be formed by assembling the plurality of light traps 33 together, and the present disclosure is not limited in this regard.

It should be noted that the light trap 33 eliminates the light emitted into the light trap cavity 331, and the eliminated light may be a weakened light or an absorption light, as long as an intensity or probability that the light is reflected into the accommodation cavity 11 can be reduced.

Referring to FIG. 19, FIG. 21, and FIG. 22, in some embodiments, the light trap cavity 331 has a conical cross section.

With such a design, after the light enters the light trap cavity 331, the light may be reflected back and forth at two sides of the cone until the light is absorbed by the cavity wall of the light trap cavity 331.

In some embodiments, a conical opening should face towards a direction in which the light emitted from the light emitting assembly 31 may enter. An angle formed between two side edges of the cone may range from 0° to 90°. This angle between the two side edges of the cone may be adjusted as desired, and the present disclosure is not limited in this regard. It can be understood that when the angle between the two side edges of the cone is small, an effect of reflecting and absorbing light is good, and when the angle between the two side edges of the cone is large, the light can easily enter the light trap cavity 331 to be reflected and absorbed by the light trap cavity 331.

It should be noted that the oil fume sensor 100 may include a plurality of light trap cavities 331. Each of the plurality of light trap cavities 331 has a conical cross section. In this case, the angle between two side edges of of the cone of each of the plurality of light trap cavities 331 may be exactly the same as or different from each other, or partially different from each other. In an embodiment, some of the plurality of light trap cavities 331 has a conical cross section, and the rest of the plurality of light trap cavities 331 are of other shapes. The embodiments of the present disclosure do not enumerate this herein.

Referring to FIG. 19, FIG. 21, and FIG. 22, in some embodiments, an angle is formed between an optical axis of the light emitting assembly 31 and an optical axis of the light receiving assembly 32. A conical vertex of the light trap cavity 331 is located on the optical axis of the light emitting assembly 31. The light trap cavities 331 are arranged symmetrically about the optical axis of the light emitting assembly 31.

The light emitted from the light emitting assembly 31 faces towards the light trap cavity 331. In this way, the light emitted into the light trap cavity 331 is continuously emitted and absorbed in the light trap cavity 331. Therefore, a better light absorption effect can be achieved.

In some embodiments, there are several angles formed between the optical axis of the light emitting assembly 31 and the optical axis of the light receiving assembly 32. These angle may be 30°, 45°, 60°, 90°, 120°, or the like, as long as the optical axis of the light emitting assembly 31 and the optical axis of the light receiving assembly 32 are not located on one straight line. The embodiments of the present disclosure do not enumerate this herein. After the light required for being received by the light receiving assembly 32 is emitted from the light receiving assembly 32, under the action of the oil fume particles 70, the light is diffracted and reflected to the light received by the light receiving assembly, and most of the remaining light emitted from the light emitting assembly 31 still moves in an emission direction of the light emitting assembly 31. Therefore, by forming the light trap cavity 331 on the optical axis of the light emitting assembly 31, the light can be absorbed better.

It can be understood that an opening of the light trap cavity 331 should face towards the light emitting assembly 31.

Referring to FIGS. 19 and 22, in some embodiments, an angle is formed between an optical axis of the light emitting assembly 31 and an optical axis of the light receiving assembly 32. A conical vertex of the light trap cavity 331 is located on the optical axis of the light receiving assembly 32, and the light trap cavities 331 are arranged symmetrically about the optical axis of the light receiving assembly 32.

Under normal conditions, a cavity wall on the optical axis of the light receiving assembly 32 can easily reflect the light to the light receiving assembly 32. Therefore, such an arrangement can effectively absorb light that would be reflected to the light receiving assembly 32, thereby reducing the error of determining the oil fumes concentration.

In some embodiments, the opening of the light trap cavity 331 should face towards the light receiving assembly 32.

Referring to FIG. 19, in some embodiments, a plurality of protruding ribs 34 of an arc-shaped section protrudes from a cavity wall of the light trap cavity 331.

Therefore, a length of the cavity wall of the light trap cavity 331 is increased. Moreover, the light can be more effectively absorbed.

In some embodiments, a plurality of protruding ribs 34 is provided. For example, there may be five, ten, fifteen, twenty, or the like protruding ribs 34, and the present disclosure is not limited in this regard.

The protruding rib 34 and the cavity wall of the light trap cavity 331 may be of an integrated structure. For example, the protruding rib 34 and the light trap 33 are integrally formed through the injection molding. In addition, for example, the protruding rib 34 and the light trap 33 are formed through simultaneous cutting. Therefore, the protruding rib 34 and the light trap 33 can be manufactured simply.

The protruding rib 34 and the cavity wall of the light trap cavity 331 may be separately arranged. For example, the protruding rib 34 is bonded to the light trap 33. For another example, the protruding rib 34 is engaged on the light trap 33. In this way, the number and arrangement of the protruding ribs 34 on each light trap 33 can be adjusted as required. As a result, manufacturing and application of the protruding rib 34 is more flexible.

There are several arrangement of the plurality of protruding ribs 34 at the cavity wall of the light trap cavity 331. For example, the plurality of protruding ribs 34 may cover the cavity wall of the light trap cavity 331 together. In addition, the plurality of protruding ribs 34 may be evenly arranged at the cavity wall of the light trap cavity 331, and a gap is formed between every two adjacent protruding ribs 34. Additionally, the plurality of protruding ribs 34 may be unevenly arranged at the cavity wall of the light trap cavity 331. The present disclosure is not limited in this regard.

It can be understood that a protruding rib 34 may protrude from the cavity wall of the light trap cavity 331 and has a trapezoidal cross section, a triangular cross section, or the like, and the detailed description thereof will be omitted herein.

In some embodiments, referring to FIG. 19, a semicircular protruding protion 1101 may protrude from the cavity wall of the accommodation cavity 11, to enhance an absorption effect of the cavity wall of the accommodation cavity 11 on the light.

In some embodiments, roughness of a cavity wall of the light trap cavity 331 is greater than or equal to 12.5 microns.

Therefore, the rough cavity wall of the light trap cavity 331 can absorb the light better.

In some embodiments, the roughness of the cavity wall of the light trap cavity 331 may be 12.5 microns, 13 microns, 15 microns, or the like. The embodiments do not enumerate this herein.

In some embodiments, a cavity wall of the light trap cavity 331 is black.

In this way, absorption efficiency of the light trap cavity 331 for the light can be effectively improved.

In some embodiments, the black is one among all colors that has the best light absorption effect. Therefore, by providing the cavity wall of the light trap cavity 331 of the black color, it is possible to better absorb the light at each wavelength.

Referring to FIG. 20 and FIG. 21, in some embodiments, a region of the accommodation cavity 11 located between the air inlet 21 and the air outlet 22 is provided with a detection cavity 29. An optical axis of the light emitting assembly 31 passes through the detection cavity 29, and an optical axis of the light receiving assembly 32 passes through the detection cavity 29.

In this way, the light receiving assembly 32 can easily receive the light influenced by the oil fume particles 70. Therefore, the detection for the oil fume particles 70 is more sensitive.

In some embodiments, the air inlet 21 may be opposite to the air outlet 22. That is, a center of the air inlet 21 and a center of the air outlet 22 are located on one straight line. In this case, the detection cavity 29 may be of a cylindrical shape. In this way, after the gas containing the oil fume particles 70 enter the accommodation cavity 11 from the air inlet 21, the gas can be quickly discharged from the air outlet 22, thereby lowering the probability that the gas containing the oil fume particles 70 escapes in the accommodation cavity 11. Therefore, the probability that the oil fume particles 70 are agglutinated in the accommodation cavity 11 and contaminate the accommodation cavity 11 is reduced. The optical axis of the light receiving assembly 32 and the optical axis of the light emitting assembly 31 may both pass through an axis of the detection cavity 29. In this way, the light receiving assembly 32 can receive light more conveniently.

Further, referring to FIG. 21, the light emitting assembly 31 includes a light emitter 311 and a transmitting lens 312. The light emitter 311 is located at a focal point of the transmitting lens 312, and light emitted from the light emitter 311 is incident to the detection cavity 29 by the transmitting lens 312.

Therefore, the light emitted from the light emitter 311 is changed by the transmitting lens 312 and can be transmitted in the form of parallel light, thereby providing more uniform detection.

In some embodiments, an emitter accommodation cavity for accommodating the light emitter 311 may be formed by the upper cover, the bottom housing, and the transmitting lens 312 together. The light emitter 311 can emit light to the detection cavity 29 through the transmitting lens 312. The transmitting lens 312 can block the oil fumes in the detection cavity 29 outside the emitter accommodation cavity, and prevent the light emitter 311 from being not in contact with the oil fumes particles 70. Therefore, it is possible to prevent the oil fumes particles 70 from being in contacting the light emitter 311 to contaminate the light emitter 311. Therefore, the service life of the light emitter 311 is prolonged.

It can be understood that the transmitting lens 312 is a convex lens.

Referring to FIG. 21, in some embodiments, the light receiving assembly 32 includes a light receiver 321 and a receiving lens 322. The light receiver 321 is located at a focal point of the receiving lens 322, and is configured to receive light by the receiving lens 322.

With this arrangement, after the light received by the light receiver 321 is shaped by the receiving lens 322, the light in each direction can be converged on the focal point of the receiving lens 322, i.e., on a position at which the light receiver 321 is located. Therefore, a receiving range of the light receiver 321 is increased without changing the light receiver 321. Moreover, sensitivity of the light receiver 321 to light changes is improved.

In some embodiments, a receiver accommodation cavity for accommodating the light receiver 321 may be formed by the upper cover, the bottom housing, and the receiving lens 322 together. The light receiver 321 can receive the light of the detection cavity 29 through the receiving lens 322. The receiving lens 322 can block the oil fumes of the detection cavity 29 outside the receiver accommodation cavity and prevent the light receiver 321 from being not in contact with the oil fume particles 70. Therefore, it is possible to prevent the oil fume particles 70 from being in contact with the light emitter 311 to contaminate the light emitter 311. Therefore, the service life of the light receiver 321 is prolonged.

Referring to FIG. 23, embodiments of the present disclosure further provide a kitchen appliance 1000. The kitchen appliance 1000 includes, but is not limited to, a range hood, an integrated cooker, or other electric appliances having an oil fume discharge function. The kitchen appliance 1000 includes a casing 200, a fan assembly 300, and an oil fume sensor 100. The fan assembly 300 is mounted in the casing 200. The oil fume sensor 100 is mounted at the casing 200.

In the kitchen appliance 1000 according to the embodiments of the present disclosure, by providing the light trap 33, the light trap cavity 331 is formed. In this way, the light emitted to the light trap cavity 331 can be reflected in the conical light trap cavity 331 several times, and is finally absorbed by the light trap 33. As a result, it is possible to reduce a probability that the light receiving assembly 32 receives the light reflected by the cavity wall of the accommodation cavity 11, and effectively eliminate the error of detecting the oil fumes concentration by the light receiving assembly 32.

In some embodiments, the kitchen appliance 1000 according to the embodiments of the present disclosure includes, but is not limited to, a deflector assembly and a check valve. The casing 200 is arranged on the deflector assembly. The deflector assembly has a smoke collecting cavity and the plurality of function buttons. The oil mesh and the top plate are provided in the smoke collecting cavity, and the plurality of function buttons may be used for the user to input the operation instruction. The fan assembly 300 is provided in the casing 200. The fan assembly 300 includes a volute and a fan, and has an air inlet and an air outlet. The fan is arranged in the volute. The volute air duct is formed in the volute. The air inlet is configured to provide the oil fumes for entering the fan assembly 300. The air inlet is configured to allow the oil fumes to enter the fan assembly 300. The air outlet is in communication with the volute air duct and is configured to allow the oil fumes to be discharged out of the fan assembly 300. The check valve is connected to the top of the casing 200. The check valve internally has a check valve air duct. It can be understood that the check valve refers to a valve that an openable/closable member is a circular valve clack and is configured to block backflow of a medium by means of its own weight and a pressure of the medium. The check valve may be a lifting check valve and a swing check valve.

In some embodiments, the air outlet 22 of the oil fume sensor 100 faces towards the air inlet of the fan assembly 300, and the air inlet 21 of the oil fume sensor 100 is spaced apart from the air inlet of the fan assembly 300.

In this way, a pressure difference is generated between the air outlet 22 and the air inlet 21 of the oil fume sensor 100 after the fan assembly 300 operates. Therefore, the airflow containing the oil fumes enters the air inlet cavity due to the pressure difference.

In some embodiments, the air inlet 21 of the oil fume sensor 100 is spaced apart from the air inlet of the fan assembly 300. However, by the fan assembly 300, the air inlet 21 of the oil fume sensor 100 may have a predetermined negative pressure such as -5Pa relative to the atmospheric pressure. By the fan assembly 300, compared with the atmospheric pressure, the air outlet 22 of the oil fume sensor 100 may have a negative pressure of about - 30Pa. In this way, a pressure difference of approximately 25Pa is formed between the air inlet 21 and the air outlet 22, thereby driving the movement of the gas containing the oil fumes.

In the description of this specification, descriptions with reference to the terms "an embodiment," "some embodiments," "illustrative embodiments," "examples," "specific examples," or "some examples" etc., mean that specific features, structure, materials or characteristics described in conjunction with the embodiment or example are included in at least an embodiment or example of the present disclosure. In this specification, the schematic representations of the above terms do not necessarily refer to the same embodiment or example. Moreover, the described specific features, structures, materials or characteristics may be combined in any one or more embodiments or examples in a suitable manner.

Although the embodiments of the present disclosure have been shown and described above, it can be understood that the above-mentioned embodiments are exemplary and should not be construed as limiting the present disclosure. Those of ordinary skill in the art can make changes, modifications, substitutions and modifications to the above-mentioned embodiments within the scope of the present disclosure.

## Claims

1. An oil fume sensor, comprising:
a housing having an accommodation cavity; and
a sensor assembly located in the accommodation cavity.

2. The oil fume sensor according to claim 1, wherein the housing has a first side wall and a second side wall, the second side wall being tightly fitted to an outer side of the first side wall to allow the accommodation cavity to be formed into an enclosed space.

3. The oil fume sensor according to claim 2, wherein the second side wall is in an interference fit with the outer side of the first side wall to allow a tight fit between the second side wall and the outer side of the first side wall.

4. The oil fume sensor according to claim 2, wherein:
the sensor assembly comprises a main control board;
the accommodation cavity comprises a first receiving cavity, the main control board being received in the first receiving cavity; and
the housing comprises an exhaust cover and an upper cover, wherein:
the exhaust cover comprises a first cover body and a first exhaust pipe penetrating the first cover body, the first exhaust pipe having a sealing groove that is formed at an end of the first exhaust pipe; and
the upper cover comprises a second cover body and a second exhaust pipe penetrating the second cover body, the second exhaust pipe being provided with a sealing protrusion engaged in the sealing groove, wherein an interior of the first exhaust pipe is in communication with an interior of the second exhaust pipe to form an exhaust cavity, and wherein the first receiving cavity is formed by the first cover body, the second cover body, the first exhaust pipe, and the second exhaust pipe.

5. The oil fume sensor according to claim 4, wherein:
the first side wall is formed at an outer side wall of the sealing protrusion; and
the second side wall is formed at an inner side wall of the sealing groove.

6. The oil fume sensor according to claim 4, wherein:
the second side wall is formed at an inner side wall of the sealing protrusion; and
the first side wall is formed at an outer side wall of the sealing groove.

7. The oil fume sensor according to claim 4, wherein:
the upper cover is provided with a hollow protector protruding from the upper cover, the second side wall being formed at an inner edge of the protector; and
the main control board is provided with a socket protruding from the main control board, the first side wall being formed at a periphery of the socket.

8. The oil fume sensor according to claim 2, wherein:
the sensor assembly comprises a light emitter, a light receiver, and a lens;
the accommodation cavity comprises a second receiving cavity, the light emitter and the light receiver being received in the second receiving cavity; and
the housing comprises an upper cover and a bottom housing, the bottom housing comprising a first housing and a first air inlet pipe penetrating the first housing, and the second receiving cavity being formed by the upper cover, the first housing, and the lens.

9. The oil fume sensor according to claim 8, wherein:
the first side wall is formed at a peripheral wall of the upper cover; and
the first housing is provided with a protruding ring, the second side wall being formed at an inner side wall of the protruding ring.

10. The oil fume sensor according to claim 8, wherein:
the housing further comprises an exhaust cover having a receiving groove, the second side wall being formed at an inner side wall of the receiving groove; and
the first housing is provided with a protruding ring, the first side wall being formed at an outer side wall of the protruding ring.

11. The oil fume sensor according to claim 8, wherein:
the housing further comprises a fixing portion configured to restrict a position of the lens; and
the first side wall is formed at an outer side wall of the lens, and the second side wall is formed at an inner side wall of the fixing portion; and/or the second side wall is formed at an inner side wall of the lens, and the first side wall is formed at an outer side wall of the fixing portion.

12. The oil fume sensor according to claim 1, wherein:
the housing further has an air inlet and an air outlet that are each in communication with the accommodation cavity; and
the housing comprises a lower housing and an air inlet pipe penetrating the lower housing, the air inlet pipe internally having an air inlet cavity, the air inlet being formed at a position communicating between the air inlet cavity and the accommodation cavity, and the air inlet cavity being tapered towards the air inlet.

13. The oil fume sensor according to claim 12, wherein the air inlet cavity is in a circular truncated cone shape.

14. The oil fume sensor according to claim 12, wherein:
the sensor assembly comprises a light emitting assembly and a light receiving assembly;
a mounting portion is disposed in the accommodation cavity, a second receiving cavity being formed by the mounting portion and the housing, the light emitting assembly and/or the light receiving assembly being mounted in the second receiving cavity; and
the housing has a pressurizing channel having an end in communication with the second receiving cavity and another end in communication with an external environment.

15. The oil fume sensor according to claim 14, wherein:
the lower housing comprises a bottom housing and an air inlet housing, the second receiving cavity being formed by the bottom housing and the mounting portion;
the air inlet pipe comprises a first air inlet pipe penetrating the bottom housing and a second air inlet pipe penetrating the air inlet housing, an interior of the first air inlet pipe being in communication with an interior of the second air inlet pipe to form the air inlet cavity; and
the bottom housing has an opening, and the air inlet housing has a third through hole, the opening being in communication with the third through hole to form the pressurizing channel.

16. The oil fume sensor according to claim 15, wherein an oil reduction cavity is formed by an outer wall of the second air inlet pipe, an inner wall of the air inlet housing, and the bottom housing together, the opening and the third through hole being both in communication with the oil reduction cavity.

17. The oil fume sensor according to claim 14, wherein the housing is internally provided with a plurality of fins arranged at intervals in the second receiving cavity along an optical axis of the light emitting assembly and/or an optical axis of the light receiving assembly, each of the plurality of fins having a light-transmitting hole.

18. The oil fume sensor according to claim 12, wherein the housing further comprises an upper housing and an exhaust pipe penetrating the upper housing, the accommodation cavity being formed by the upper housing and the lower housing together, the exhaust pipe internally having an exhaust cavity, the air outlet being formed at a position communicating between the exhaust cavity and the second receiving cavity.

19. The oil fume sensor according to claim 18, wherein an axis of the air inlet cavity and an axis of the exhaust cavity are located on a straight line.

20. The oil fume sensor according to claim 1, wherein a detection cavity is formed at a position where the accommodation cavity contacts oil fumes, the detection cavity having a inclined guide surface and a through hole that is formed at a lower cavity wall of the detection cavity, and the inclined guide surface being configured to guide an oil drop to be discharged from the detection cavity through the through hole.

21. The oil fume sensor according to claim 20, wherein:
the through hole comprises a first through hole;
the housing has an air inlet and an air outlet that are both in communication with the detection cavity;
the inclined guide surface is formed at the lower cavity wall of the detection cavity; and
the air inlet serves as the first through hole.

22. The oil fume sensor according to claim 21, wherein the through hole further comprises a second through hole formed at the lower cavity wall of the detection cavity and spaced apart from the first through hole.

23. The oil fume sensor according to claim 20 or 21, wherein an inclination angle of the inclined guide surface relative to a horizontal plane is greater than or equal to 10°.

24. The oil fume sensor according to claim 21, wherein the inclined guide surface comprises a first inclined guide surface formed at an upper cavity wall of the detection cavity.

25. The oil fume sensor according to claim 24, wherein:
the guide inclined surface comprises a second inclined guide surface;
the air outlet is located at the upper cavity wall of the detection cavity, and a guide protrusion protrudes from a periphery of the air outlet, the second inclined guide surface being formed at a periphery of the guide protrusion; and
an inclination angle of the second guide inclined surface is greater than the inclination angle of the first guide inclined surface.

26. The oil fume sensor according to claim 20, wherein:
the sensor assembly comprises a light emitting assembly and a light receiving assembly; and
a mounting portion is disposed in the accommodation cavity, a receiving cavity being formed by the mounting portion and the housing, the light emitting assembly and/or the light receiving assembly being mounted in the receiving cavity, the receiving cavity having an opening formed at a lower cavity wall of the receiving cavity, and the opening serving as the through hole.

27. The oil fume sensor according to claim 26, wherein the housing comprises a plurality of fins arranged at intervals in the receiving cavity along an optical axis of the light emitting assembly and/or an optical axis of the light receiving assembly, each of the plurality of fins having a light-transmitting hole and a liquid flowing groove in communication with the light-transmitting hole, and the inclined guide surface being formed onat a wall of the liquid flowing groove.

28. The oil fume sensor according to claim 27, wherein:
the light emitting assembly comprises a light emitter and a transmitting lens, wherein the transmitting lens is mounted in several spaces between the plurality of fins, the receiving cavity comprises a first cavity and a second cavity, the first cavity being spaced apart from the second cavity by the transmitting lens, the light emitter being mounted in the second cavity, and the opening comprises a first opening formed at a lower cavity wall of the first cavity; and/or
the light receiving assembly comprises a light receiver and a receiving lens, wherein the receiving lens is mounted in several spaces between the plurality of fins, the receiving cavity comprises a third cavity and a fourth cavity, the third cavity being spaced apart from the fourth cavity by the receiving lens, the light receiver being mounted in the fourth cavity, and the opening comprises a second opening formed at a lower cavity wall of the third cavity.

29. The oil fume sensor according to claim 28, wherein:
the opening comprises a third opening formed at a lower cavity wall of the second cavity wall; and/or
the opening comprises a fourth opening formed at a lower cavity wall of the fourth cavity wall.

30. The oil fume sensor according to claim 1, wherein:
the housing comprises an air inlet and an air outlet that are in communication with the accommodation cavity;
a light emitting assembly is configured to emit light to the accommodation cavity;
a light receiving assembly is configured to receive the light emitted from the light emitting assembly; and
a plurality of light traps provided in the accommodation cavity, each of the plurality of light traps having a light trap cavity configured to remove light emitted into the light trap cavity.

31. The oil fume sensor according to claim 30, wherein the light trap cavity has a conical cross section.

32. The oil fume sensor according to claim 31, wherein:
an angle is formed between an optical axis of the light emitting assembly and an optical axis of the light receiving assembly; and
a conical vertex of the light trap cavity is located on the optical axis of the light emitting assembly, and the light trap cavities are arranged symmetrically about the optical axis of the light emitting assembly.

33. The oil fume sensor according to claim 31 or 32, wherein:
an angle is formed between an optical axis of the light emitting assembly and an optical axis of the light receiving assembly; and
a conical vertex of the light trap cavity is located on the optical axis of the light receiving assembly, and the light trap cavities are arranged symmetrically about the optical axis of the light receiving assembly.

34. The oil fume sensor according to claim 30, wherein a plurality of protruding ribs having an arc-shaped cross section protrude from a cavity wall of the light trap cavity.

35. The oil fume sensor according to claim 30, wherein roughness of a cavity wall of the light trap cavity is greater than or equal to 12.5 microns.

36. The oil fume sensor according to claim 30, wherein a cavity wall of the light trap cavity is black.

37. The oil fume sensor according to claim 30, wherein a region of the accommodation cavity located between the air inlet and the air outlet is provided with a detection cavity, an optical axis of the light emitting assembly and an optical axis of the light receiving assembly both passing through the detection cavity.

38. The oil fume sensor according to claim 37, wherein the light emitting assembly comprises a light emitter and a transmitting lens, the light emitter being located at a focal point of the transmitting lens, and light emitted from the light emitter being incident to the detection cavity through the transmitting lens.

39. The oil fume sensor according to claim 37, wherein the light receiving assembly comprises a light receiver and a receiving lens, the light receiver being located at a focal point of the receiving lens and being configured to receive light through the receiving lens.

40. A kitchen appliance, comprising:
a casing;
a fan assembly mounted in the casing; and
an oil fume sensor according to any one of claims 1 to 39.

41. The kitchen appliance according to claim 40, wherein the oil fume sensor is arranged at an air inlet of the fan assembly.
